(19) Europäisches Patentamt
European Patent Office
Office européen des brevets

(11) Publication number: **0 099 673**
**A2**

(12) **EUROPEAN PATENT APPLICATION**

(21) Application number: 83303765.8

(22) Date of filing: 29.06.83

(51) Int. Cl.³: C 07 C 177/00
A 61 K 31/557, C 08 B 37/16
C 07 D 307/93

(30) Priority: 30.06.82 JP 112757/82

(43) Date of publication of application:
01.02.84 Bulletin 84/5

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Wakatsuka, Hirohisa
3-3-708, Miyano-cho
Takatsuki-shi Osaka(JP)

(72) Inventor: Yamato, Takashi
15-9-3-303, Tsukahara 1-chome
Takatsuki-shi Osaka(JP)

(72) Inventor: Hashimoto, Shinsuke
2-6-905, Shirakawa, 3-chome
Ibaraki-shi Osaka(JP)

(74) Representative: Bentham, Stephen et al,
J.A. Kemp & Co. 14 South Square Gray's Inn
London WC1R 5EU(GB)

(54) **Prostaglandin D derivatives, process for their preparation and compositions containing them.**

(57) Prostaglandin D derivatives of the general formula:

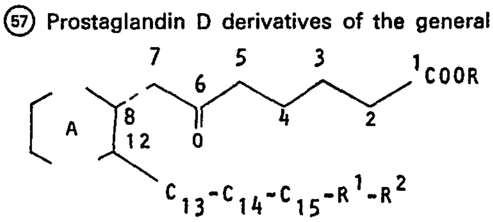

(I)

wherein [A] represents a group of the formula:

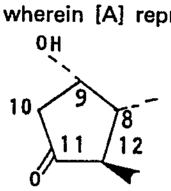

(II)

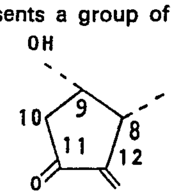

(III)

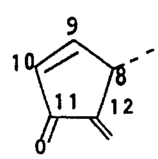

(IV)

$C_{13}$-$C_{14}$-$C_{15}$ represents
(i) a group of the formula:

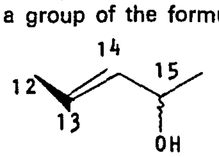

(V)

when [A] represents a group of the formula (II), or

(ii) a group of the formula:

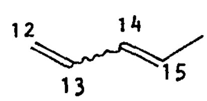

(VI)

when [A] represents a group of the formula (III) or (IV), R represents hydrogen, alkyl of 1 - 12 carbon atoms, $R^1$ represents a single bond or alkylene of 1 - 5 carbon atoms, $R^2$ represents alkyl of 1 - 8 carbon atoms, cycloalkyl of 4 - 7 carbon atoms optionally substituted by alkyl of 1 - 8 carbon atoms, or represents phenyl or phenoxy optionally substituted by halogen, trifluoromethyl or alkyl of 1 - 4 carbon atoms; the double bond between $C_9$ - $C_{10}$ in formula (IV) is *Z*, the double bond between $C_{13}$ - $C_{14}$ in formula (V) is *E*, and the double bonds between $C_{12}$ - $C_{13}$ and between $C_{14}$ - $C_{15}$ in formula (VI) are *E*, *Z* or a mixture thereof, with the proviso that when $R^1$ represents a single bond, $R^2$ does not represent phenoxy; and when R represents hydrogen, non-toxic salts thereof and cyclodextrin clathrates thereof; possess anti-tumor activity.

EP 0 099 673 A2

0099673



"PROSTAGLANDIN D DERIVATIVES, PROCESS FOR THEIR PREPARATION AND COMPOSITIONS CONTAINING THEM"

The present invention relates to novel prostaglandin D analogues, to processes for their preparation and pharmaceutical compositions containing them.

Prostaglandins are derivatives of prostanoic acid having the following structure:

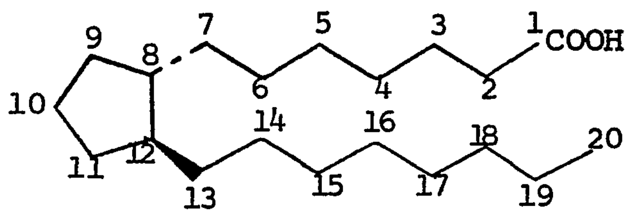

Various types of prostaglandins are known, and their types depend on the structure of the alicyclic ring and the substituents. For example, the alicyclic rings of prostaglandins F (PGF), E (PGE) and D (PGD) have the following structures,

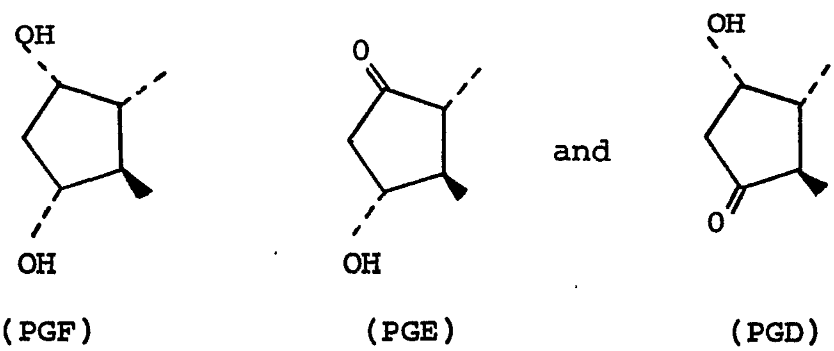

In the above structural formulae or in the other structural formulae in this specification, according to the generally accepted nomenclature, the dotted line indicates that the substituent attached thereto is behind the ring

plane, i.e., is of the α-configuration, the bold line indicates that the substituent attached thereto is in front of the ring plane, i.e., is of the β-configuration, and the wavy line indicates that the substituent attached thereto is of the α-configuration or the β-configuration or a mixture thereof.

These compounds are sub-classified according to the positions of the double bonds in the side chains attached to the alicyclic ring at the 8-position and the 12-position. The PG-1 compound has a <u>trans</u> double bond (<u>trans</u>-$\Delta^{13}$) between $C_{13}$-$C_{14}$ and the PG-2 compound has a <u>cis</u> double bond between $C_5$ - $C_6$ and a <u>trans</u> double bond between $C_{13}$ - $C_{14}$ (<u>cis</u>-$\Delta^5$ , <u>trans</u>-$\Delta^{13}$). For example, prostaglandin $D_1$ ($PGD_1$) and prostaglandin $D_2$ ($PGD_2$) may be expressed by the following structural formulae, respectively:

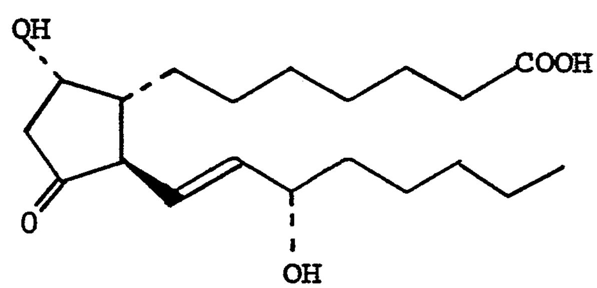

($PGD_1$)

and

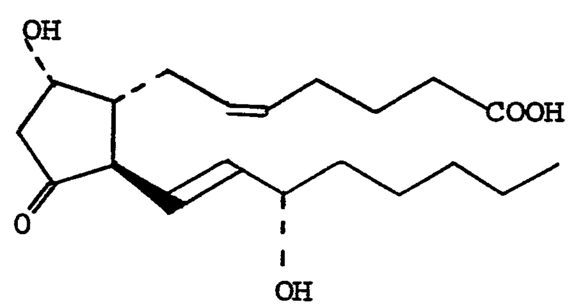

($PGD_2$)

Further, when one or more methylene groups are removed from the aliphatic group attached at the 12-position of the alicyclic ring of a prostaglandin, said compound is known as a nor-prostaglandin according to the general rule of the organic nomenclature, and the number of the removed methylene groups is indicated by adding di-, tri-, etc. before the prefix "nor".

The prostaglandins generally have pharmacological properties. For example, they exert various effects including the stimulation of contraction of smooth muscles, a hypotensive effect, a diuretic effect, a bronchial dilation effect, the inhibition of lypolysis, the inhibition of platelet aggregation and the inhibition of gastric acid secretion. Therefore, they are useful in treatments of hypertension, thrombosis, asthma and gastric and intestinal ulcers, in the induction of labor and abortion in pregnant mammals, in the prevention of arteriosclerosis and also as diuretics. They are liposoluble substances present in extremely small quantities in the tissues which secrete prostaglandins in vivo in animals.

As a result of research and experimentation to discover novel compounds which have the pharmacological effects of the "natural" prostaglandins, or which have one or more of these properties to an enhanced degree, or which have properties which are not found in the "natural" prostaglandins, it has been found that novel $PGD_1$ analogues

in which an oxo group (i.e. =0) has been introduced onto the carbon atom at the 6-position of $PGD_1$ and certain derivatives thereof (6-keto-$PGD_1$ derivatives), further novel PGD derivatives in which, in addition, the hydroxy group attached to the carbon atom at the 15-position of these compounds has been removed to introduce double bonds between $C_{12}$ - $C_{13}$ and between $C_{14}$- $C_{15}$, and further novel PGD derivatives in which the hydroxy group attached to the carbon atom at the 9-position of these compounds has been removed to introduce a <u>cis</u>-double bond between $C_9$ - $C_{10}$ have a surprisingly strong anti-tumor effect whereas they have no or only very weak pharmacological properties possessed by the "natural" prostaglandins.

Heretofore, there have been filed several patent applications relating to PGD analogues. For example, compounds wherein various substituents have been introduced into the side chain attached to the 12-position are disclosed in the specification of United States Patent No. 4,016,184 (Derwent No. 24680Y), and further compounds in which the carboxy group at the 1-position of PGD analogues has been replaced by various functional groups are disclosed in the specifications of United States Patent Nos. 4,346,228, 4,028,419, 4,055,602, 4,032,576, 4,123,463 and British Patent Nos. 1554041 and 1554042; see Derwent Nos. 81216X, 43349Y, 48794Y, 48793Y, 81996A, 46957Y and 46957Y, respectively. However, none of the above

applications discloses the 6-keto-$PGD_1$ derivatives of the present invention which include, of course, such derivatives wherein a hydroxy group at the 15-position or both hydroxy groups at the 9- and 15-positions have been removed to introduce one or two double bonds.

Furthermore, in addition to their structural differences from known PGD compounds, the compounds of the present invention have an effect which has not hitherto been found in the known PGD analogues, that is, an anti-tumor effect. The above-described Patent specifications describe only the pharmacological properties known with the natural prostaglandins and none of the applications refers to an anti-tumor effect.

The present invention accordingly provides prostaglandin D derivatives of the general formula:

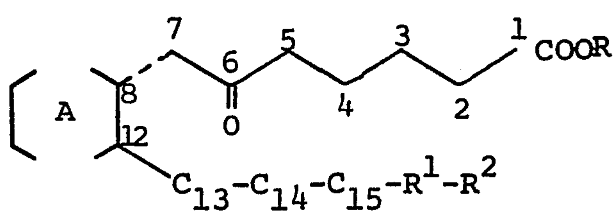

(I)

wherein [A] represents a group of the formula:

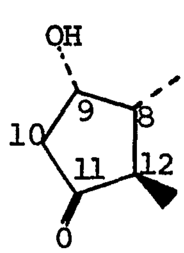

(II)

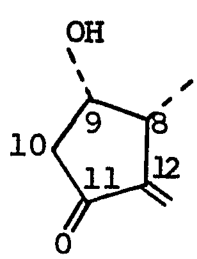

(III)

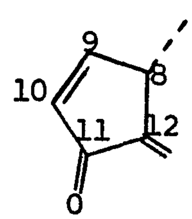

(IV)

$C_{13}$-$C_{14}$-$C_{15}$ represents

(i) a group of the formula:

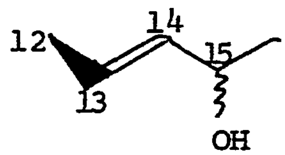

(V)

when [A] represents a group of the formula (II), or

(ii) a group of the formula:

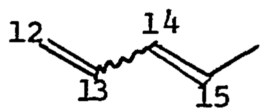

(VI)

when [A] represents a group of the formula (III) or (IV), R represents a hydrogen atom or a straight-chain or branched-chain alkyl group of 1 - 12 carbon atoms, $R^1$ represents a single bond or a straight-chain or branched-chain alkylene group of 1 - 5 carbon atoms, $R^2$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms, or represents a phenyl or phenoxy group either unsubstituted or substituted by at least one halogen atom, trifluoromethyl group or straight-chain or branched-chain alkyl group of 1 - 4 carbon atoms, the double bond between $C_9$ - $C_{10}$ in formula (IV) is <u>Z</u>, the double bond between $C_{13}$ - $C_{14}$ in formula (VI) is <u>E</u>, and the double bonds between $C_{12}$ - $C_{13}$ and between $C_{14}$ - $C_{15}$ in formula (VI), which may be in the same or different configurations, are

E, Z or a mixture thereof (i.e., EZ), with the proviso that when $R^1$ represents a single bond, $R^2$ does not represent a substituted or unsubstituted phenoxy group; and, when [A] represents a group of formula (II) or (III), mixtures of the prostaglandin derivatives of general formula (I) and their tautomers; and, when R represents a hydrogen atom, non-toxic salts thereof; and cyclodextrin clathrates thereof.

The compounds of the present invention include 6-keto-$PGD_1$ derivatives of the general formula:

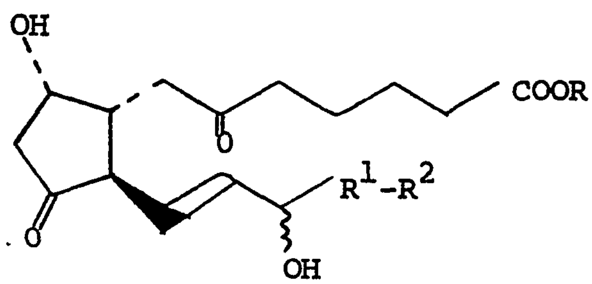

(Ia)

(wherein the various symbols are as hereinbefore defined); 6-keto-$PGD_1$ derivatives of the general formula:

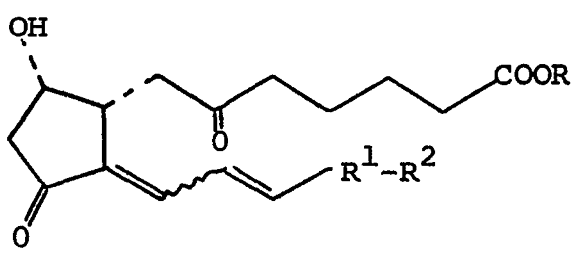

(Ib)

(wherein the various symbols are as hereinbefore defined), and 6-keto-$PGD_1$ derivatives of the general formula:

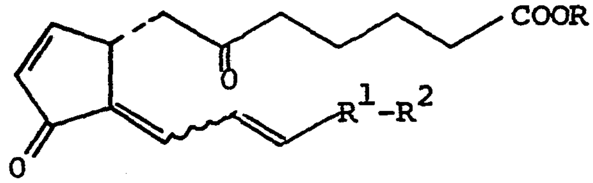

(Ic)

wherein the various symbols are as hereinbefore defined.

In the compounds of the general formula (I), there are several asymmetric carbons. For example, in the compounds of the general formula (Ia), there are four asymmetric carbons (that is, carbon atoms at the 8-, 9-, 12- and 15-positions); in the compounds of the general formula (Ib), the carbon atoms at the 8- and 9-positions are asymmetric carbons; and in the compounds of the general formula (Ic), the carbon atom at the 8-position is asymmetric. Further asymmetric carbon atoms may occur in the groups represented by R, $R^1$ and $R^2$. The presence of asymmetric carbon atoms leads to the existence of isomerism. It is to be understood that all isomers and mixtures thereof arising from the presence of asymmetric carbon atoms in the compounds of the invention are to be considered within the scope of general formula (I).

It will be understood by one skilled in the art that each of the compounds represented by general formula (Ia) and by general formula (Ib) may exist in an equilibrium state with its respective tautomer, i.e., the compound represented by the general formula (Ia') and the compound represented by the general formula (Ib'), respectively.

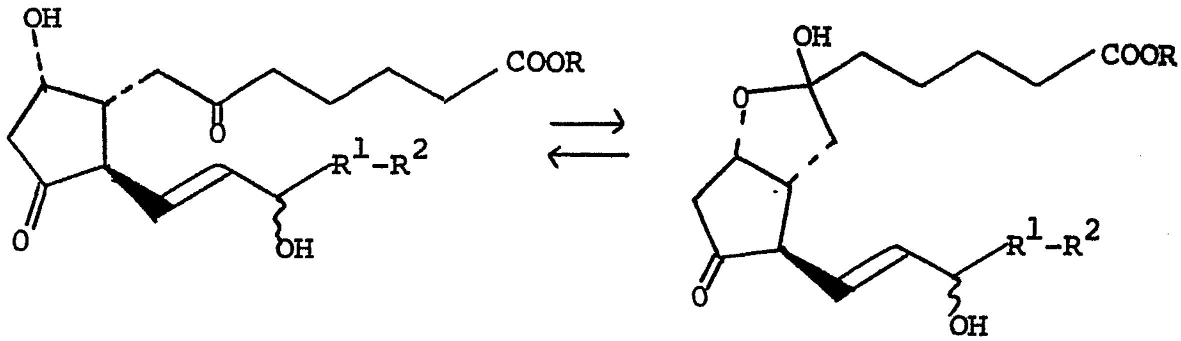

(Ia) (Ia')

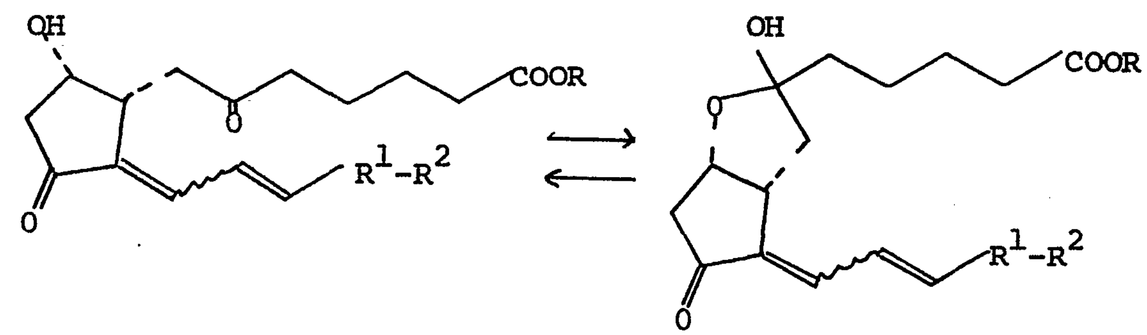

(Ib) (Ib')

wherein the various symbols are as hereinbefore defined and, in general formulae (Ia') and (Ib'), the absolute configuration of the 6-position is R or S or a mixture thereof (i.e. RS).

It is to be understood that the tautomeric forms of formula (Ia') and (Ib'), and mixtures thereof with the PGD derivatives of formula (Ia) and (Ib), respectively, are within the scope of the present invention and that a reference in this specification or the accompanying claims to PGD derivatives of general formula (I) embraces the tautomeric forms thereof.

In the general formula (I), the alkyl group of 1 - 12 carbon atoms represented by R includes methyl, ethyl, propyl, butyl, pentyl, hexyl, heptyl, octyl, nonyl, decyl, undecyl and dodecyl groups and isomers thereof; R preferably represents a hydrogen atom or an alkyl group of 1 - 4 carbon atoms, and more especially a hydrogen atom or a methyl or ethyl group.

In the general formula (I), the alkylene group of 1 - 5 carbon atoms represented by $R^1$ includes methylene, ethylene, trimethylene, tetramethylene and pentamethylene groups and isomers thereof; $R^1$ preferably represents a single bond or a methylene or ethylene group.

In the general formula (I), the alkyl group of 1 - 8 carbon atoms represented by $R^2$ includes methyl, ethyl, propyl, butyl, pentyl, hexyl, heptyl and octyl groups and isomers thereof; $R^2$ preferably represents a butyl, pentyl or hexyl group either unsubstituted or substituted by one or two methyl or ethyl groups; pentyl and hexyl groups unsubstituted or substituted by one or two methyl groups are especially preferred.

In the general formula (I), the substituted or unsubstituted cycloalkyl group represented by $R^2$ includes cyclobutyl, cyclopentyl, cyclohexyl and cycloheptyl groups, and cyclobutyl, cyclopentyl, cyclohexyl and cycloheptyl groups substituted by one or more methyl, ethyl, propyl, butyl, pentyl, hexyl, heptyl or octyl groups; cyclobutyl,

cyclopentyl or cyclohexyl groups either unsubstituted or substituted by one methyl, ethyl, propyl or butyl group are preferred; 3-propyl-cyclopentyl is especially preferred.

In the general formula (I), the substituted or unsubstituted phenyl or phenoxy group represented by $R^2$ includes phenyl and phenoxy and phenyl and phenoxy groups substituted by one or more atoms or groups selected from fluorine atoms, chlorine atoms, trifluoromethyl groups, and methyl, ethyl, propyl and butyl groups, phenyl or phenoxy groups either unsubstituted or substituted by one chlorine atom, trifluoromethyl group, methyl or ethyl group are preferred; chlorine is a preferred substituent.

In the general formula (I), preferred groupings $R^1 - R^2$ are butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, 2-ethylheptyl, cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl, 4-butylcyclohexyl, benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl and 4-ethylphenoxymethyl; pentyl, 2-methylhexyl, 3-propylcyclopentyl, benzyl and 3-chlorophenoxymethyl are especially preferred.

In general formula (I) when $C_{13}$-$C_{14}$-$C_{15}$ represents a group of the formula (V), the preferred configuration of the hydroxyl group attached to the carbon atom at the 15-position is the α-configuration.

According to a feature of the present invention, the prostaglandin D derivatives of general formula (Ic) wherein the various symbols are as hereinbefore defined can be prepared by reacting a 6-keto-$PGD_1$ derivative of general formula (Ia), wherein the various symbols are as hereinbefore defined, with an aqueous acid, preferably in an inert solvent, for example, methylene chloride, chloroform, carbon tetrachloride, diethyl ether, N,N-dimethylformamide, tetrahydrofuran, or a mixture of two or more such solvents, using as the aqueous acid, for example, an aqueous solution of an inorganic acid such as hydrochloric acid, hydrobromic acid, sulfuric acid, and phosphoric acid or an organic acid such as acetic acid, propionic acid and oxalic acid, at the reflux temperature of the reaction mixture. The reaction is preferably carried out in tetrahydrofuran using 1N hydrochloric acid at the reflux temperature of the reaction mixture.

The compounds of the present invention of general formulae (Ia) and (Ib) wherein the various symbols are as

hereinbefore defined can be prepared by the hydrolysis, under acidic conditions, of a compound of the general formula:

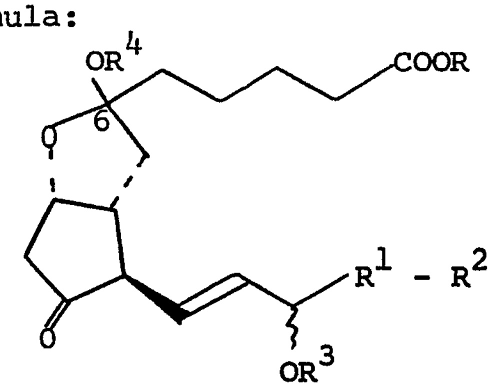

(VII)

[wherein $R^3$ represents a tetrahydropyran-2-yl group or tetrahydrofuran-2-yl group either unsubstituted or substituted by at least one alkyl group, or a 1-ethoxyethyl group, preferably a tetrahydropyran-2-yl group, $R^4$ represents a lower alkyl group, preferably a methyl group, and the other symbols are as hereinbefore defined, and the absolute configuration of the 6-position carbon atom is R or S or a mixture thereof (i.e. RS)] followed by the separation by known methods of the mixture of prostaglandin derivatives of general formulae (Ia) and (Ib) obtained. By the expression "known methods" as used in this specification is meant methods heretofore used or described in the literature. The hydrolysis under acidic conditions is conducted, for example,

(1) in an aqueous solution of an organic acid such as acetic acid, propionic acid, oxalic acid or p-toluenesulfonic acid or an aqueous solution of an inorganic acid such as hydrochloric acid, sulfuric acid or phosphoric acid,

suitably in the presence of a water-miscible organic solvent, for example, a lower alkanol such as methanol or ethanol (preferably methanol) or an ether such as 1,2-dimethoxyethane, dioxane or tetrahydrofuran (preferably tetrahydrofuran) at a temperature of from room temperature to 80°C, or

(2) in an anhydrous lower alkanol such as methanol or ethanol in the presence of an organic acid such as p-toluenesulfonic acid or trifluoroacetic acid at a temperature of 10 - 45°C. The hydrolysis is preferably conducted using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran, or a mixture of p-toluenesulfonic acid and anhydrous methanol.

The mixture of prostaglandin derivatives of formulae (Ia) and (Ib) obtained can be separated into the derivatives of general formula (Ia) and the derivative of general formula (Ib) by conventional separation means, for example, thin layer or column chromatography using silica gel or high performance liquid chromatography.

According to a further feature of the present invention the prostaglandin derivatives of general formula (Ic) may also be prepared by reacting a compound of general formula (Ib) or (VII), wherein the various symbols are as

hereinbefore defined, with an aqueous acid as hereinbefore described for the preparation of a prostaglandin derivative of general formula (Ic) from a prostaglandin derivative of general formula (Ia).

The compound of general formula (VII), wherein the various symbols are as hereinbefore defined, can be prepared by oxidizing to an oxo group the hydroxy group attached to the 11-position of a compound of the general formula:

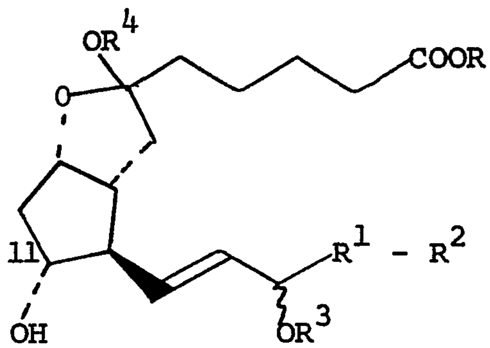

(VIII)

wherein the various symbols are as hereinbefore defined and the absolute configuration at the 6-position is R or S or a mixture thereof (i.e. RS). Such an oxidation reaction is well known, and is described in detail in, for example,

(a) "Organic Synthetic Chemistry III, Synthesis Part 1", edited by Tetsuji Kameya, published by Nanko-do, page 176 - 206 (August 1, 1976), or

(b) "Compendium of Organic Synthetic Methods" published by John Wiley & Sons, Inc. (U.S.A.), Vol. 1 (1971), Vol. 2 (1974) and Vol. 3 (1977), Section 48 or 168.

The oxidation is preferably carried out under mild neutral conditions using, for example, a dimethylsulphide-N-chlorosuccinimide complex, a thioanisole-N-chlorosuccinimide complex, a dimethylsulphide-chlorine complex, a thioanisole-chlorine complex [for these complexes, see J. Amer. Chem. Soc., 94, 7586 (1972)], a dicyclohexylcarbodiimide-dimethylsulphoxide complex [see J. Amer. Chem. Soc., 87, 5661 (1965)], pyridinium chlorochromate ($C_5H_5NH$-$CrO_3Cl$) [see Tetrahedron Letters, 2647 (1975)], a sulphuric anhydride-pyridine complex [see J. Amer. Chem. Soc., 89, 5505 (1967)], chromyl chloride [see J. Amer. Chem. Soc., 97, 5929 (1975)], a chromium trioxide-pyridine complex (e.g. Collins' reagent), Jones' reagent or chromic acid solution (prepared from chromium trioxide, manganese sulphate, sulphuric acid and water), oxalyl chloride and dimethylsulphoxide (i.e. the Swern oxidation); suitably Collins' reagent, Jones' reagent or the Swern reagent is employed. The oxidation reaction using Collins' reagent is conducted in a halogenated hydrocarbon such as chloroform, methylene chloride or carbon tetrachloride at a temperature of from room temperature to 0°C, preferably at 0°C. The oxidation reaction using Jones' reagent is generally conducted at a temperature below room temperature. The oxidation reaction using Swern reagent is conducted in a halogenated hydrocarbon such as chloroform or methylene chloride at a

temperature of from -50°C to -60°C, and then treatment with triethylamine.

The compound of general formula (VIII) can be produced by introducing an alkoxy group (i.e. a group $OR^4$) into the 5-position of a compound of the general formula:

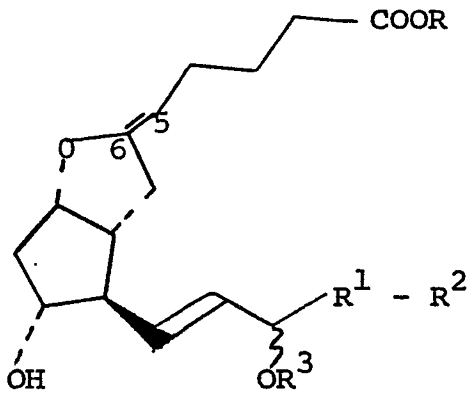

(IX)

wherein the various symbols are as hereinbefore defined and the double bond between $C_5$ - $C_6$ is Z. This reaction is conducted in a lower alkanol corresponding to $R^4$, such as methanol or ethanol, at a temperature of 0 to 50°C, preferably at room temperature using an organic acid, for example, acetic acid, propionic acid or oxalic acid, or an inorganic acid, for example, hydrochloric acid. It is necessary to carry out the reaction carefully to avoid hydrolysis of the group $OR^3$.

The compound of general formula (IX) can be produced by subjecting a compound of the general formula:

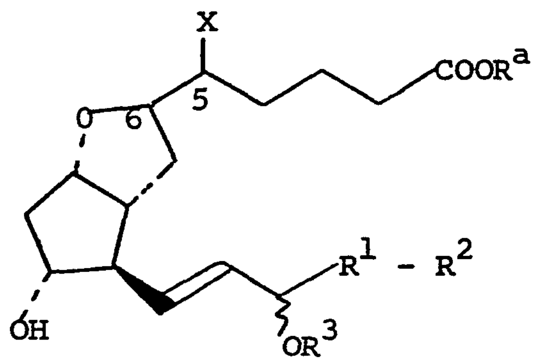

(X)

(wherein $R^a$ represents a straight-chain or branched-chain alkyl group of 1 - 12 carbon atoms, X represents an iodine atom or a bromine atom and the other symbols are as hereinbefore defined, and the absolute configurations of the 5- and 6-positions are independently R or S or a RS mixture) to a dehydrohalogenation reaction and then, if desired, subjecting the resulting ester to saponification to produce the corresponding carboxylic acid.

Reagents used for the dehydrohalogenation are well known and include, for example, bicyclic amines such as 1,5-diazabicyclo[5.4.0]undecene-5 (DBU), 1,5-diazabicyclo[4.3.0]nonene-5 (DBN) or 1,4-diazabicyclo[2.2.0]-octane (DABCO), or alkali metal alcoholates, for example, a lower alcoholate of sodium or potassium. The reaction is effected at a temperature of from room temperature to 110°C, preferably from room temperature to 80°C; when an amine is used the reaction may be carried out in the presence or absence of a solvent, preferably in toluene or in the absence of solvent; when an alcoholate is used, the reaction is preferably carried out in a corresponding lower alkanol.

The compound of general formula (IX) wherein R represents a hydrogen atom and the other symbols are as hereinbefore defined can be produced by saponifying by known methods a compound of general formula (IX) wherein R represents an alkyl group of 1 - 12 carbon atoms and the other symbols are as hereinbefore defined. The saponification is preferably conducted using an aqueous solution of a hydroxide of an alkali metal such as sodium, potassium or lithium, or of an alkaline earth metal such as calcium or barium, in a lower alkanol of 1 - 4 carbon atoms such as methanol or ethanol, or in a mixture of a lower alkanol of 1 - 4 carbon atoms and an ether such as dioxane or tetrahydrofuran at -10°C to room temperature, preferably at room temperature.

The compound of general formula (IX) wherein R represents a hydrogen atom obtained by saponification is preferably presented to the subsequent reaction after it is isolated and purified in a sodium form thereof using the preparation of salts hereinafter described.

The compound of general formula (X) can be produced by subjecting a compound of the general formula:

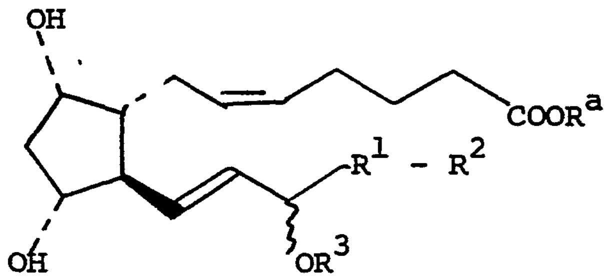

(XI)

wherein the various symbols are as hereinbefore defined, to a simultaneous halogenation and cyclization reaction, which may be conducted in the following manner: (1) When X in the compound of general formula (X) represents an iodine atom, the reaction is effected using iodine in pyridine at a temperature of from room temperature to 0°C or using iodine in an inert organic solvent such as methylene chloride or chloroform in the presence of a carbonate of an alkali metal such as sodium or potassium or in the presence of a bicarbonate of an alkali metal at a temperature of from room temperature to 0°C, or (2) when X in the compound of general formula (X) represents a bromine atom, the reaction is effected using N-bromosuccinimide or N-bromoacetamide in a non-protonic organic solvent, for example, methylene chloride, chloroform, carbon tetrachloride, diethyl ether, N,N-dimethylformamide, tetrahydrofuran or a mixture thereof at a temperature of -30 - 70°C. Preferably, the reaction is effected by adding a saturated aqueous sodium bicarbonate solution to a methylene chloride solution of the compound of general formula (XI) and adding slowly and dropwise a methylene

chloride solution of iodine while vigorously stirring the resulting two layers at 0°C.

The compound of general formula (XI) can be produced by the sequence of reaction steps illustrated by the following scheme. In the scheme, $R^5$ represents a straight-chain or branched-chain alkyl group of 1 - 4 carbon atoms or a phenyl group, preferably an n-butyl group or a phenyl group, and the other symbols are as hereinbefore defined.

Scheme

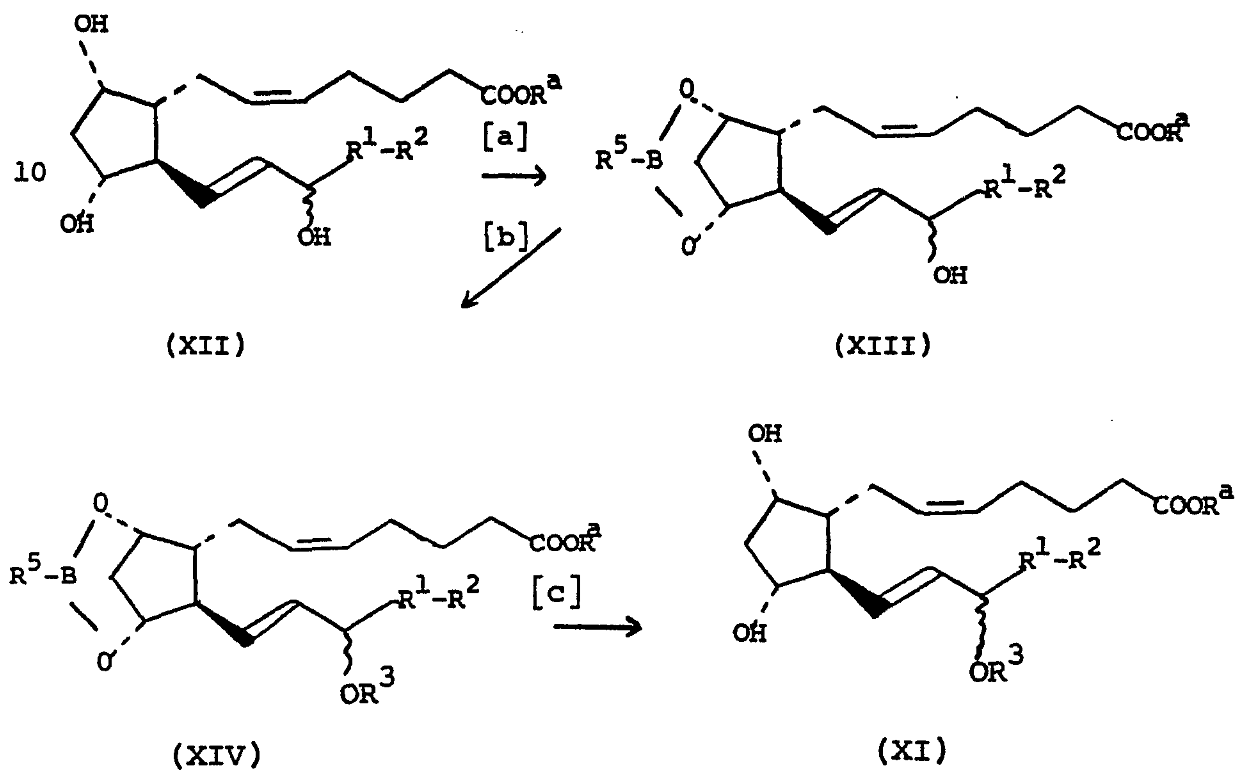


(XII) (XIII)

(XIV) (XI)

All of the steps in the scheme can be conducted by known reactions. For example, the step [a] can be conducted by reacting with a boric acid corresponding to $R^5$, for example, phenylboric acid [$PhB(OH)_2$] or butylboric acid [$n\text{-}BuB(OH)_2$], in an inert organic solvent such as methylene chloride, chloroform, carbon tetrachloride or tetrahydrofuran in the presence of molecular sieves 3A or 4A at reflux temperature [see J.C.S. Chem. Comm., page 658 (1975) and Prostaglandins, Vol. 9, page 109 (1975)].

The step [b] can be conducted using e.g. 2,3-dihydropyran, 2,3-dihydrofuran, ethyl vinyl ether, in an inert organic solvent, for example, methylene chloride, chloroform or diethyl ether, in the presence of a condensing agent, for example, p-toluenesulphonic acid, sulfuric acid or trifluoroacetic acid at a temperature of from room temperature to -30°C. Preferably, it is conducted using 2,3-dihydropyran in methylene chloride in the presence of pyridinium p-toluenesulfonate or p-toluenesulfonic acid at room temperature.

The step [c] can be conducted by reacting with an aqueous hydrogen peroxide solution in an aqueous solution of an alkanol of 1 - 4 carbon atoms, for example, aqueous methanol or ethanol, at a temperature of not higher than 60°C, preferably at 45 - 50°C.

The products of steps [a], [b] and [c] can be used, without purification, in subsequent steps.

In the sequence of the reaction steps illustrated by the above scheme, the compound of the general formula (XII) employed as the starting material is known as a $PGF_2$ derivative and can be produced by converting the $OR^3$ groups at the 11-position and the 15-position of a compound of the general formula:

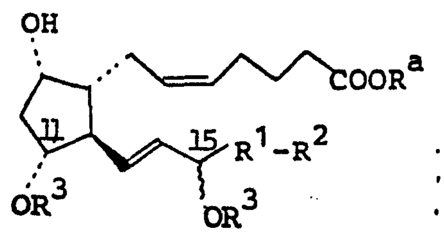

(XIII)

wherein the various symbols are as hereinbefore defined, to hydroxy groups under the reaction conditions employed in the hydrolysis of compounds of general formula (VII) to compounds of general formulae (Ia) and (Ib).

The compounds of general formula (XIII) can be prepared by the application or adaptation of known methods, for example:

(1) when the grouping $-R^1-R^2$ represents a pentyl group, as described in J. Amer. Chem. Soc., 91, 5675 (1969) or ibid, 92, 397 (1970);

(2) when the grouping $-R^1-R^2$ represents an alkyl group, as described in Japanese Patent Kokai Nos. 42675/72, 54068/73, 64073/73, 124048/74, 95250/75, 96543/75 and 101340/75, British Patent Specification Nos. 1398291, 1483240 and 1540427, United States Patent Specification

No. 4024174, and Belgian Patent No. 850084;

(3) when $R^2$ of the grouping $-R^1-R^2$ represents a cycloalkyl group, as described in Japanese Patent Kokai Nos. 109353/74, 95250/75, 96543/75, 123647/75, 148339/75, 122040/76, 125256/76, 27753/77 and 25544/78, British Patent Specification Nos. 1464916, 1488141, 1483240, 1484210 and 1545213, United States Patent Specifications Nos. 3966792, 4034003, 4024174, 4045468 and 4087620, and Belgian Patent No. 844256;

(4) when $R^2$ of the grouping $-R^1-R^2$ represents a phenyl group or a phenoxy group, as described in Japanese Patent Kokai Nos. 95250/75, 96543/75, 59841/76, 101961/76 and 25745/77, British Patent Specifications Nos. 1483240 and 1521747, United States Patent Specification Nos. 4024174 and 4065632, and Belgian Patent No. 845348.

Cyclodextrin clathrates of the prostaglandin D derivatives of general formula (I) can be prepared by known methods using α-, β- or γ-cyclodextrin or a mixture thereof, for example using the process described in the specifications of United States Patent Nos. 3,816,393 and 4,054,736. Conversion into their cyclodextrin clathrates serves to increase the stability of the prostaglandin D derivatives of the invention.

The prostaglandin derivatives of general formula (I) wherein R represents a hydrogen atom may, if desired, be converted by known methods into salts. Preferably the

salts are non-toxic salts. By the term "non-toxic salts" as used in this specification is meant salts the cations of which are relatively innocuous to the animal organism when used in therapeutic doses so that the beneficial pharmacological properties of the compound of general formula (I) are not vitiated by side effects caused by these cations. The salts are preferably water-soluble. Suitable salts include for example salts of alkali metals such as sodium or potassium, salts of alkaline earth metals such as calcium or magnesium, ammonium salts and pharmaceutically acceptable (non-toxic) amine salts. Amines suitable for forming such salts with a carboxylic acid are well known and include, for example, amines derived in theory by the replacement of one or more of the hydrogen atoms of ammonia by groups, which may be the same or different when more than one hydrogen atom is replaced, selected from, for example, alkyl groups containing from 1 to 6 carbon atoms and hydroxyalkyl groups containing 2 or 3 carbon atoms. Suitable non-toxic amine salts are, e.g., tetraalkylammonium, such as tetramethylammonium, salts, and other organic amine salts such as methylamine salts, ethylamine salts, isopropylamine salts, <u>tert</u>-butylamine salts, dimethylamine salts, cyclopentylamine salts, benzylamine salts, phenethylamine salts, piperidine salts, monoethanolamine salts, diethanolamine salts, lysine salts or arginine salts.

Salts may be prepared from the acids of general formula (I) wherein R represents a hydrogen atom, by known methods, for example by reaction of stoichiometric quantities of an acid of general formula (I) and the appropriate base, e.g. an alkali metal or alkaline earth metal hydroxide or carbonate, ammonium hydroxide, ammonia or an organic amine, in a suitable solvent. The salts may be isolated by lyophilisation of the solution or, if sufficiently insoluble in the reaction medium, by filtration, if necessary, after removal of part of the solvent.

The prostaglandin D derivatives of general formula (I), and, when R in general formula (I) represents a hydrogen atom, non-toxic salts thereof, and cyclodextrin clathrates thereof either do not show the pharmacological effects typical of other prostaglandins, for example, hypotensive activity, inhibition of platelet aggregation, stimulation of uterine muscles and the production of diarrhoea or these effects are very weak. In contrast the anti-tumor effect of the compounds of the invention is extremely strong and moreover their toxicity is extremely low. They may therefore be employed as very effective anti-tumor agents in the prevention or therapy of tumors, for example, leukemia and solid cancer, including treatment to produce remission thereof.

In a laboratory in vitro test on the inhibition of proliferation of human myelogenous leukemic cells (HL-60),

the compounds of the present invention showed an inhibiting effect. The experimental method and the results are described below.

Test on Inhibition of Proliferation Using Human Myelogenous Leukemic Cells (HL-60)

<Experimental Method>

The test on the inhibition of proliferation of human myelogenous leukemic cells was conducted by a well-known method. The human myelogenous leukemic cells (HL-60) were added to an RPMI culture solution containing 10% bovine fetal serum, the number of cells in the culture solution was adjusted to 1 x $10^5$ cells/ml, a solution in ethanol of the compound under test was added to give a concentration of 5 μg/ml, and the mixture was subjected to stationary culture at 37°C for 4 days. As a control, a culture solution to which 0.1% of ethanol had been added was similarly cultured. After the cultivation, the culture solutions were stained by the Trypan Blue staining method and the surviving cell number was measured to determine the percentage inhibition relative to the control.

The results are given in the following Table.

TABLE

Inhibition of Proliferation Using Human Myelogenous Leukemic Cells (HL-60)

| Compound | Surviving Cell Number (cells/ml) | Percentage Inhibition (%) |
|---|---|---|
| Control | $29.4 \times 10^4$ | - |
| 6-Keto-$PGD_1$ methyl ester | 0 | 100 |
| (12EZ,14EZ)-(9α)-6,11-Dioxo-9-hydroxyprosta-12,14-dienoic acid methyl ester | 0 | 100 |
| (9Z,12EZ,14EZ)-6,11-Dioxoprosta-9,12,14-trienoic acid methyl ester | 0 | 100 |

The acute toxicity of the compounds of the present invention was low, so that the 6-keto-PGD derivatives of the present invention may be considered to be sufficiently safe and suitable for medical use.

Preferred prostaglandin D derivatives of general formula (I) are, for example, as follows:

6-keto-$PGD_1$ methyl ester, 16-methyl-6-keto-$PGD_1$ methyl ester, 16,16-dimethyl-6-keto-$PGD_1$ methyl ester, 17,20-dimethyl-6-keto-$PGD_1$ methyl ester, 15-(1-butylcyclobutyl)-16,17,18,19,20-pentanor-6-keto-$PGD_1$ methyl ester, 15-cyclopentyl-16,17,18,19,20-pentanor-6-keto-$PGD_1$ methyl ester, 15-(3-propylcyclopentyl)-16,17,18,19,20-pentanor-6-keto-$PGD_1$ methyl ester, 15-(4-butylcyclohexyl)-16,17,18,19,20-pentanor-6-keto-$PGD_1$ methyl ester, 16-phenyl-17,18,19,20-tetranor-6-keto-$PGD_1$ methyl ester,

16-phenoxy-17,18,19,20-tetranor-6-keto-$PGD_1$ methyl ester, 16-(3-chlorophenoxy)-17,18,19,20-tetranor-6-keto-$PGD_1$ methyl ester, 16-(3-trifluoromethylphenoxy)-17,18,19,20-tetranor-6-keto-$PGD_1$ methyl ester, (12EZ,14EZ)-(9α)-6,11-dioxo-9-hydroxyprosta-12,14-dienoic acid methyl ester, (12EZ,14EZ)-(9α)-6,11-dioxo-9-hydroxy-16-methylprosta-12,14-dienoic acid methyl ester, (12EZ,14EZ)-(9α)-6,11-dioxo-9-hydroxy-16,16-dimethylprosta-12,14-dienoic acid methyl ester, (12EZ,14EZ)-(9α)-6,11-dioxo-9-hydroxy-17,20-dimethylprosta-12,14-dienoic acid methyl ester, (12EZ,14EZ)-(9α)-6,11-dioxo-9-hydroxy-15-(1-butylcyclobutyl)-16,17,18,19,20-pentanorprosta-12,14-dienoic acid methyl ester, (12EZ,14EZ)-(9α)-6,11-dioxo-9-hydroxy-15-cyclopentyl-16,17,-18,19,20-pentanorprosta-12,14-dienoic acid methyl ester, (12EZ,14EZ)-(9α)-6,11-dioxo-9-hydroxy-15-(3-propylcyclopentyl)-16,17,18,19,20-pentanorprosta-12,14-dienoic acid methyl ester, (12EZ,14EZ)-(9α)-6,11-dioxo-9-hydroxy-15-(4-butylcyclohexyl)-16,17,18,19,20-pentanorprosta-12,14-dienoic acid methyl ester, (12EZ,14EZ)-(9α)-6,11-dioxo-9-hydroxy-16-phenyl-17,18,19,20-tetranorprosta-12,14-dienoic acid methyl ester, (12EZ,14EZ)-(9α)-6,11-dioxo-9-hydroxy-16-phenoxy-17,18,19,20-tetranorprosta-12,14-dienoic acid methyl ester,

(12EZ,14EZ)-(9α)-6,11-dioxo-9-hydroxy-16-(3-chlorophenoxy)-17,18,19,20-tetranorprosta-12,14-dienoic acid methyl ester,

(12EZ,14EZ)-(9α)-6,11-dioxo-9-hydroxy-16-(3-trifluoromethylphenoxy)-17,18,19,20-tetranorprosta-12,14-dienoic acid methyl ester,

(9Z,12EZ,14EZ)-6,11-dioxoprosta-9,12,14-trienoic acid methyl ester,

(9Z,12EZ,14EZ)-6,11-dioxo-16-methylprosta-9,12,14-trienoic acid methyl ester,

(9Z,12EZ,14EZ)-6,11-dioxo-16,16-dimethylprosta-9,12,14-trienoic acid methyl ester,

(9Z,12EZ,14EZ)-6,11-dioxo-17,20-dimethylprosta-9,12,14-trienoic acid methyl ester,

(9Z,12EZ,14EZ)-6,11-dioxo-15-(1-butylcyclobutyl)-16,17,18,-19,20-pentanorprosta-9,12,14-trienoic acid methyl ester,

(9Z,12EZ,14EZ)-6,11-dioxo-15-cyclopentyl-16,17,18,19,20-pentanorprosta-9,12,14-trienoic acid methyl ester,

(9Z,12EZ,14EZ)-6,11-dioxo-15-(3-propylcyclopentyl)-16,17,-18,19,20-pentanorprosta-9,12,14-trienoic acid methyl ester,

(9Z,12EZ,14EZ)-6,11-dioxo-15-(4-butylcyclohexyl)-16,17,18,-19,20-pentanorprosta-9,12,14-trienoic acid methyl ester,

(9Z,12EZ,14EZ)-6,11-dioxo-16-phenyl-17,18,19,20-tetranorprosta-9,12,14-trienoic acid methyl ester,

(9Z,12EZ,14EZ)-6,11-dioxo-16-phenoxy-17,18,19,20-tetranorprosta-9,12,14-trienoic acid methyl ester,

(9Z,12EZ,14EZ)-6,11-dioxo-16-(3-chlorophenoxy)-17,18,19,20-tetranorprosta-9,12,14-trienoic acid methyl ester,

(9Z,12EZ,14EZ)-6,11-dioxo-16-(3-trifluoromethylphenoxy)-17,18,19,20-tetranorprosta-9,12,14-trienoic acid methyl ester

and the corresponding ethyl esters thereof, and the corresponding carboxylic acids thereof (i.e. prostaglandin derivatives of general formula (I) wherein R represents a hydrogen atom).

The following Reference Examples and Examples illustrate the preparation of compounds of the present invention. In the Reference Examples and Examples, 'TLC', 'IR', 'NMR' and 'Mass' represent 'Thin layer chromatography', 'Infrared absorption spectrum', 'Nuclear magnetic resonance' and 'Mass spectrum', respectively. Where solvent ratios are specified in chromatographic separations, the ratios are by volume: the solvents in parentheses in thin layer chromatography show the developing solvents used. Except when specified otherwise, infrared spectra are recorded by the liquid film method and nuclear magnetic resonance spectra are recorded in deuterochloroform ($CDCl_3$) solution.

## Reference Example 1

Methyl (5Z,13E)-(9α,11α,15S)-9,11-dihydroxy-15-(tetrahydropyran-2-yloxy)-prosta-5,13-dienoate.

Molecular sieve 4A (20 - 25 grains) were added to a solution of 1 g. of $PGF_{2\alpha}$ methyl ester and 409 mg. of phenylboric acid in 25 ml. of dry methylene chloride, and the solution was refluxed for 1 hr. After cooling to room temperature, the reaction mixture was filtered. To the filtrate, 0.382 ml. of 2,3-dihydropyran and 70.3 ml. of pyridinium p-toluenesulphonate were added, and the reaction mixture was then neutralized with a saturated aqueous solution of sodium bicarbonate. The oily layer obtained was separated off, washed successively with water

and a saturated aqueous solution of sodium chloride, dried over anhydrous magnesium sulphate, and concentrated under reduced pressure. The residue obtained was dissolved in 10 ml. of methanol, and to the solution obtained 40 ml. of water and 4 ml. of a 30% aqueous solution of hydrogen peroxide were added, and the solution was heated to 45 - 48°C. After cooling to room temperature, the solution was concentrated under reduced pressure. To the residue 50 ml. of ethyl acetate was added. The solution obtained was washed successively with water and a saturated aqueous solution of sodium chloride, dried over anhydrous magnesium sulphate, and concentrated under reduced pressure. The residue obtained was purified by column chromatography on silica gel using a mixture of cyclohexane and ethyl acetate as eluent, to obtain 913 mg. of the title compound having the following physical characteristics:

TLC (benzene:ethyl acetate = 1:2): Rf = 0.41;

NMR: $\delta$ = 5.63-5.16(4H,m), 4.71(1H,m), 3.66(3H,s), 4.24-3.35(5H.m), 0.88(3H,t);

IR: $\nu$ = 3440, 2940, 1740, 1432 $cm^{-1}$;

Mass:m/e = 434, 421, 406.

The following compounds were obtained by the same procedure as Reference Example 1:

(a) Methyl (5Z,13E)-(9α,11α,15S,17S)-9,11-dihydroxy-15-(tetrahydropyran-2-yloxy)-17,20-dimethylprosta-5,13-dienoate

Starting material: 17S,20-dimethyl-$PGF_2\alpha$ methyl ester;

TLC (benzene:ethyl acetate = 1:2): Rf = 0.43;

NMR: $\delta$ = 5.7-5.2(4H,m), 4.7(1H,m), 3.6(3H,s), 4.2-3.3(5H,m), 1.0-0.8(6H,m);

IR: $\nu$ = 3440, 2938, 1737 $cm^{-1}$;

Mass:m/e = 462.

(b) Methyl (5Z,13E)-(9α,11α,15S)-9,11-dihydroxy-15-(tetrahydropyran-2-yloxy)-15-(3-propylcyclopentyl)-16,17,18,19,20-pentanorprosta-5,13-dienoate

Starting material: 15-(3-propylcyclopentyl)-16,17,18,19,20-pentanor-$PGF_{2\alpha}$ methyl ester;

TLC (benzene:ethyl acetate = 1:2): Rf = 0.45;

NMR: $\delta$ = 5.7-5.2(4H,m), 4.7(1H,m), 3.7(3H,s), 4.2-3.3(5H,m), 0.9(3H,t);

IR: $\nu$ = 3440, 2935, 1735 $cm^{-1}$;

Mass:m/e = 474.

(c) Methyl (5Z,13E)-(9α,11α,15S)-9,11-dihydroxy-15-(tetrahydropyran-2-yloxy)-16-phenyl-17,18,19,20-tetranorprosta-5,13-dienoate

Starting material: 16-phenyl-1,7,18,19,20-tetranor-$PGF_{2\alpha}$ methyl ester

TLC (benzene:ethyl acetate = 1:2): Rf = 0.40;

NMR:$\delta$ = 7.5-7.1(5H,m), 5.7-5.2(4H,m), 4.7(1H,m), 3.6(3H,s), 4.2-3.3(5H,m)

IR: $\nu$ = 3350, 2937, 1737 $cm^{-1}$;

Mass:m/e = 454.

(d) Methyl (5<u>Z</u>,13<u>E</u>)-(9$\alpha$,11$\alpha$,15<u>R</u>)-9,11-dihydroxy-15-(tetrahydropyran-2-yloxy)-16-(3-chlorophenoxy)-17,18,19,20-tetranorprosta-5,13-dienoate

Starting material: 16-(3-chlorophenoxy)-17,18,19,20-tetranor-$PGF_{2\alpha}$ methyl ester;

TLC (benzene:ethyl acetate = 1:2): Rf = 0.40;

NMR:$\delta$ = 7.3-6.7(4H,m), 5.7-5.2(4H,m), 4.7-3.8(8H,m), 3.7(3H,s);

IR: $\nu$ = 3440, 2935, 1737, 1600 $cm^{-1}$;

Mass: m/e = 504.

## Reference Example 2

**Methyl (13E)-(5RS,6RS,9$\alpha$,11$\alpha$,15S)-5-iodo-6,9-epoxy-11-hydroxy-15-(tetrahydropyran-2-yloxy)prosta-13-enoate**

To a solution of 700 mg of $PGF_{2\alpha}$ derivative (prepared in Reference Example 1) in 15 ml. of methylene chloride, 15 ml. of a saturated aqueous solution of sodium bicarbonate was added and the mixture was stirred vigorously at 0°C. To the solution, a solution of 432 mg. of iodine in 15 ml. of methylene chloride was added

over a period of 40 minutes with stirring, and the resulting solution was stirred for 20 minutes at the same temperature. The reaction mixture was diluted with diethyl ether, and the diluted solution was washed successively with water and a saturated aqueous solution of sodium chloride, dried over anhydrous magnesium sulphate, and concentrated under reduced pressure. The residue was purified by column chromatography on silica gel using a mixture of n-hexane and ethyl acetate as eluent, to obtain 815 mg of the title compound having the following physical characteristics:

TLC (ethyl acetate:n-hexane = 2:1): Rf = 0.35;

NMR: $\delta$ = 5.8-5.2(2H,m), 4.8-4.3(2H,m), 3.66(3H,s), 1.0-0.7(3H,m);

IR: $\nu$= 3460, 2930, 1735, 1435 $cm^{-1}$;

Mass:m/e = 476, 458, 432.

The following compounds were obtained by the same procedure as Reference Example 2:

(a) Methyl (5<u>RS</u>,6<u>RS</u>,9α,11α,15<u>S</u>,17<u>S</u>)-5-iodo-6,9-epoxy-11-hydroxy-15-(tetrahydropyran-2-yloxy)-17,20-dimethylprost-13-enoate

Starting material: $PGF_{2\alpha}$ derivative prepared in Reference Example 1(a):

TLC (ethyl acetate:n-hexane = 2:1): Rf = 0.36;

NMR: $\delta$ = 5.6-5.3(2H,m), 4.8-4.3(2H,m), 3.68(3H,s), 1.0-0.86(6H,m);

IR: $\nu$ = 3470, 2940, 1738, 1020 $cm^{-1}$;

Mass:m/e = 504, 486.

(b) Methyl (13E)-(5RS,6RS,9α,11α,15S)-5-iodo-6,9-epoxy-11-hydroxy-15-(tetrahydropyran-2-yloxy)-15-(3-propylcyclopentyl)-16,17,18,19,20-pentanorprost-13-enoate

Starting material: $PGF_2$ derivative prepared in Reference Example 1(b);

TLC (ethyl acetate:n-hexane = 2:1): Rf = 0.36;

NMR:δ = 5.8-5.2(2H,m), 4.8-4.4(2H,m), 3.66(3H,s), 0.88(3H, t);

IR: $\nu$ = 3460, 2930, 1735 $cm^{-1}$;

Mass:m/e = 516, 498.

(c) Methyl (13E)-(5RS,6RS,9α,11α,15S)-5-iodo-6,9-epoxy-11-hydroxy-15-(tetrahydropyran—2-yloxy)-16-phenyl 17,18,19,20-tetranorprost-13-enoate

Starting material: $PGF_{2\alpha}$ derivative prepared in Reference Example 1(c);

TLC (ethyl acetate:n-hexane = 2:1): Rf = 0.34;

NMR: δ = 7.5-7.1(5H,m), 5.7-5.2(2H,m), 4.8-4.3(2H,m), 3.66(3H,s);

IR: $\nu$ = 3460, 2935, 1737 $cm^{-1}$;

Mass:m/e = 496, 478.

(d) Methyl (13E)-(5RS,6RS,9α,11α,15R)-5-iodo-6,9-epoxy-11-hydroxy-15-(tetrahydropyran-2-yloxy)-16-(3-chlorophenoxy)-17,18,19,20-tetranorprost-13-enoate

Starting material: $PGF_{2\alpha}$ derivative prepared in Reference Example 1(d);

TLC (ethyl acetate:n-hexane = 2:1): Rf = 0.33;

NMR: δ = 7.3-6.7(dH,m), 5.8-5.2(2H,m), 4.8-4.3(2H,m);

IR: ν = 3460, 2930, 1736, 1600 $cm^{-1}$;

Mass:m/e = 546, 528.

Reference Example 3

Methyl (5Z,13E)-(9α,11α,15S)-6,9-epoxy-11-hydroxy-15-(tetrahydropyran-2-yloxy)prosta-5,13-dienoate

In an atmosphere of nitrogen, 1 ml. of 1,5-diazabicyclo)5.4.0]undecene-5 was added to a solution of 300 mg. of 5-iodo compound (prepared in Reference Example 2) in 1 ml of toluene, and the solution obtained was stirred for 18 hours. The solution was then diluted with diethyl ether, and the diluted solution was washed with water, dried over anhydrous magnesium sulphate and concentrated under reduced pressure to obtain 231 mg. of crude title compound having the following physical characteristics. The compound obtained was used in the reaction of Reference Example 4 without purification.

TLC (ethyl acetate:n-hexane = 2:1): Rf = 0.47;

IR: ν = 3450, 2930, 1735, 1690 $cm^{-1}$;

Mass:m/e = 450, 432, 419.

The following compounds were obtained by the same procedure as Reference Example 3:

(a) Methyl (5Z,13E)-(9α,11α,15S,17S)-6,9-epoxy-11-hydroxy-15-(tetrahydropyran-2-yloxy)-17,20-dimethylprosta-5,13-dienoate

Starting material: 5-iodo compound prepared in Reference Example 2(a);

TLC (ethyl acetate:n-hexane = 2:1): Rf = 0.48;

IR: $\nu$ = 3440, 2935, 1738, 1698 $cm^{-1}$

Mass:m/e = 478, 460, 447.

(b) Methyl (5Z,13E)-(9α,11α,15S)-6,9-epoxy-11-hydroxy-15-(tetrahydropyran-2-yloxy)-15-(3-propylcyclopentyl)-16,17,18,19,20-pentanorprosta-5,13-dienoate

Starting material: 5-iodo compound prepared in Reference Example 2(b);

TLC (ethyl acetate:n-hexane = 2:1): Rf = 0.48;

IR: $\nu$ = 3450, 2930, 1736, 1692 $cm^{-1}$;

Mass:m/e = 490, 472, 459.

(c) Methyl (5Z,13E)-(9α,11α,15S)-6,9-epoxy-11-hydroxy-15-(tetrahydropyran-2-yloxy)-16-phenyl-17,18,19,20-tetranorprosta-5,13-dienoate

Starting material: 5-iodo compound prepared in Reference Example 2(c);

TLC (ethyl acetate:n-hexane = 2:1): Rf = 0.45;

IR: $\nu$ = 3450, 2932, 1737, 1692 $cm^{-1}$;

Mass:m/e = 470, 452, 439.

(d) Methyl (5Z,13E)-(9α,11α,15R)-6,9-epoxy-11-hydroxy-15-(tetrahydropyran-2-yloxy)-16(3-chlorophenoxy)-17,18,19,20-tetranorprosta-5,13-dienoate

Starting material: 5-iodo compound prepared in Reference Example 2(d);

TLC (ethyl acetate:n-hexane = 2:1): Rf = 0.45;

IR: $\nu$ = 3450, 2932, 1736, 1693, 1600 $cm^{-1}$;

Mass:m/e = 520, 502, 489.

Reference Example 4

Methyl (13E)-(6RS,9α,11α,15S)-6-methoxy-6,9-epoxy-11-hydroxy-15-(tetrahydropyran-2-yloxy)prost-13-enoate

In an atmosphere of nitrogen, 0.1 ml. of acetic acid was added to a solution of 212 mg. of the methyl 6,9-epoxy-5-enoate compound (prepared in Reference Example 3) in 4 ml. of methanol, and the solution was stirred for 1 hr. at room temperature. The solution was diluted with ethyl acetate, and the diluted solution was washed successively with a saturated aqueous solution of sodium bicarbonate and a saturated aqueous solution of sodium chloride, dried over anhydrous magnesium sulphate, and concentrated under reduced pressure. The residue was purified by column chromatography on silica gel using a mixture of n-hexane and ethyl acetate as eluent, to obtain 195 mg. of the title compound having the following physical characteristics:

TLC (ethyl acetate:n-hexane = 2:1): Rf = 0.41 and 0.38;

NMR: δ = 5.7-5.2(2H,m), 4.7-4.3(2H,m), 3.68(3H,s), 3.23 and 3.14(3H, double s), 1.0-0.7(3H,m);

IR: ν = 3460, 2930, 1735, 1435 $cm^{-1}$;

Mass:m/e = 451, 420, 380.

The following compounds were obtained by the same procedure as Reference Example 4:

(a) Methyl (13E)-(6RS,9α,11α,15S,17S)-6-methoxy-6,9-epoxy-11-hydroxy-15-(tetrahydropyran-2-yloxy)-17,20-dimethylprost-13-enoate

Starting material: methyl 6,9-epoxy-5-enoate compound prepared in Reference Example 3(a);

TLC (ethyl acetate:n-hexane = 2:1): Rf = 0.42 and 0.39;

NMR: δ = 5.6-5.3(2H,m), 4.7-4.3(2H,m), 3.66(3H,s), 323 and 3.14(3H, double s), 1.00-0.87(6H,m);

IR: ν = 3470, 2930, 1738 $cm^{-1}$;

Mass:m/e = 479, 448.

(b) Methyl (13E)-(6RS,9α,11α,15S)-6-methoxy-6,9-epoxy-11-hydroxy-15-(tetrahydropyran-2-yloxy)-15-(3-propylcyclopentyl)-16,17,18,19,20-pentanorprost-13-enoate

Starting material: methyl 6,9-epoxy-5-enoate compound prepared in Reference Example 3(b);

TLC (ethyl acetate:n-hexane = 2:1): Rf = 0.42 and 0.39;

NMR: δ = 5.7-5.2(2H,m), 4.7-43.3(2H,m), 3.68(3H,s), 3.23 and 3.14 (3H, double s), 0.9(3H,t);

IR: ν = 3460, 2930, 1736 $cm^{-1}$;

Mass:m/e = 491, 460.

(c) Methyl (13E)-(6RS,9α,11α,15S)-6-methoxy-6,9-epoxy-11-hydroxy-15-(tetrahydropyran-2-yloxy)-16-phenyl-17,18,19,20-tetranorprost-13-enoate

Starting material: methyl 6,9-epoxy-5-enoate compound prepared in Reference Example 3(c);

TLC (ethyl acetate:n-hexane = 2:1): Rf = 0.40 and 0.37;

NMR: δ = 7.5-7.1(5H,m), 5.7-5.2(2H,m), 4.7-4.3(2H,m), 3.68(3H,s), 3.22 and 3.12(3H, double s),

IR: ν = 3460, 2932, 1737 $cm^{-1}$:

Mass:m/e = 471, 440.

(d) Methyl (13E)-(6RS,9α,11α,15R)-6-methoxy-6,9-epoxy-11-hydroxy-15-(tetrahydropyran-2-yloxy)-16-(3-chlorophenoxy)-17,18,19,20-tetranorprost-13-enoate

Starting material: methyl 6,9-epoxy-5-enoate compound prepared in Reference Example 3(d);

TLC (ethyl acetate:n-hexane = 2:1): Rf = 0.39 and 0.36;

NMR: δ = 7.3-6.7(4H,m), 5.7-5.3(2H,m), 4.7-4.3(2H,m), 3.67(3H ,s), 3.25 and 3.16(3H, double s),

IR: ν = 3460, 2930, 1735, 1600 $cm^{-1}$;

Mass:m/e = 521, 490.

## Reference Example 5

Methyl (13E)-(6RS,9α,15S)-6-methoxy-6,9-epoxy-11-oxo-5-(tetrahydropyran-2-yloxy)prost-13-enoate

In an atmosphere of nitrogen, 1.2 ml. of pyridine and 740 mg of chromium trioxide were added to 22 ml. of methylene chloride, and the mixture was stirred for 15 minutes. To the solution, 3.4 g of CELITE (registered trade mark; diatomaceous earth) was added and the mixture was cooled to 0°C. To the cooled mixture, a solution of 178 mg. of 11-hydroxy compound (prepared in Reference Example 4) in 3 ml. of methylene chloride was added all at once. The reaction mixture was diluted with ethyl acetate, filtered, and the filtrate was washed successively with water, a 5% aqueous solution of copper sulphate and a saturated aqueous solution of sodium chloride, dried over anhydrous magnesium sulphate, and concentrated under reduced pressure. The residue was purified by column chromatography on silica gel using a mixture of n-hexane and ethyl acetate as eluent, to obtain 130 mg of the title compound having the following physical characteristics:

TLC (n-hexane:ethyl acetate = 2:1): Rf = 0.38 and 0.35;

IR: $\nu$ = 2930, 1735, 1430 $cm^{-1}$;

Mass:m/e = 480, 449, 448.

The following compounds were obtained by the same procedure as Reference Example 5:

(a) Methyl (13E)-(6RS,9α,15S,17S)-6-methoxy-6,9-epoxy-11-oxo-15-(tetrahydropyran-2-yloxy)-17,20-dimethylprost-13-enoate

Starting material: 11-hydroxy compound prepared in Reference Example 4(a);

TLC (n-hexane:ethyl acetate = 2:1): Rf = 0.39 and 0.36;

IR: ν = 2930, 1736 $cm^{-1}$;

Mass:m/e = 476.

(b) Methyl (13E)-(6RS,9α,15S)-6-methoxy-6,9-epoxy-11-oxo-15-(tetrahydropyran-2-yloxy)-15-(3-propylcyclopentyl)-16,17,18,19,20-pentanorprost-13-enoate

Starting material: 11-hydroxy compound prepared in Reference Example 4(b);

TLC (n-hexane:ethyl acetate = 2:1): Rf = 0.39 and 0.36;

IR: ν = 2930, 1735 $cm^{-1}$;

Mass:m/e = 488.

(c) Methyl (13E)-(6RS,9α,15S)-6-methoxy-6,9-epoxy-11-oxo-(tetrahydropyran-2-yloxy)-16-phenyl-17,18,19,20-tetranorprost-13-enoate

Starting material: 11-hydroxy compound prepared in Reference Example 4(c);

TLC (n-hexane:ethyl acetate = 2:1): Rf = 0.37 and 0.34;

IR: $\nu$ = 2930, 1736 $cm^{-1}$;

Mass:m/e = 468.

(d) Methyl (13<u>E</u>)-(6<u>RS</u>,9α,15<u>R</u>)-6-methoxy-6,9-epoxy-11-oxo-15-(tetrahydropyran-2-yloxy)-16-(3-chlorophenoxy)-17,18,19,20-tetranorprost-13-enoate

Starting material: 11-hydroxy compound prepared in Reference Example 4(d);

TLC (n-hexane:ethyl acetate = 2:1): Rf = 0.36 and 0.33;

IR: $\nu$ = 2930, 1737, 1600 $cm^{-1}$;

Mass:m/e = 518.

## Example 1

(i) Methyl (13<u>E</u>)-(9α,15<u>S</u>)-6,11-dioxo-9,15-dihydroxyprost-13-enoate [i.e. 6-keto-$PGD_1$ methyl ester] and

(ii) Methyl (12<u>EZ</u>,14<u>EZ</u>)-(9α)-6,11-dioxo-9-hydroxy-prosta-12,14-dienoate

To a solution of 130 mg. of the 6,9-epoxy compound (prepared in Reference Example 5) in 0.5 ml. of tetrahydrofuran, 5 ml. of a 65% aqueous solution of acetic acid was added, and the solution was stirred for 10 minutes at 80°C. The reaction mixture was diluted with ice-water, and the diluted solution was extracted with ethyl acetate. The extract was washed successively with a saturated aqueous solution of sodium bicarbonate and a saturated aqueous solution of sodium chloride,

dried over anhydrous magnesium sulphate, and concentrated under reduced pressure. The residue was purified by column chromatography on silica gel using a mixture of n-hexane and ethyl acetate as eluent, to obtain 42 mg. of the title compound (i) and 10 mg. of the title compound (ii) having the following physical characteristics, respectively:

Title compound (i):

TLC (ethyl acetate:n-hexane = 2:1): Rf = 0.19;

NMR: $\delta$ = 5.72-5.34(2H,m), 4.98-4.72(½H,m), 4.64-4.52(½H,m), 4.20-4.01(1H,m);

IR: $\nu$ = 3450, 2930, 1730, 1715, 1435 $cm^{-1}$;

Mass:m/e = 364, 346, 333.

Title compound (ii):

TLC (ethyl acetate:n-hexane = 2:1): Rf = 0.47;

NMR:$\delta$ = 7.02-6.86(1H,m), 6.35-6.02(2H,m), 5.00-4.80(1H,m), 3.84-3.65(1H,m), 3.65(3H,s), 1.0-0.8(3H,m);

IR: $\nu$ = 3450, 2930, 1730, 1710, 1620, 1600, 1200, 975 $cm^{-1}$;

Mass:m/e = 364($M^+$), 346, 333.

The following compounds were obtained by the same procedure as Example 1:

(a) (i) Methyl (13<u>E</u>)-(9α,15<u>S</u>,17<u>S</u>)-6,11-dioxo-9,15-dihydroxy-17,20-dimethylprost-13-enoate [i.e. 17S,20-dimethyl-6-keto-PGD methyl ester] and

(ii) Methyl (12EZ,14EZ) - (9α,17S)-6,11-dioxo-9-hydroxy-17,20-dimethylprosta-12,14-dienoate

Starting material: 6,9-epoxy compound prepared in Reference Example 5(a);

Title compound (i):

TLC (ethyl acetate:n-hexane = 2:1): Rf = 0.20;

NMR: δ = 5.7-5.36(2H,m), 5.00-4.75(½H,m), 4.58(½H,m), 4.20-4.02(1H,m), 3.68(3H,s), 1.00-0.89(6H,m);

IR: ν = 3470, 2940, 1737 $cm^{-1}$;

Mass:m/e = 392, 374, 361.

Title compound (ii)

TLC (ethyl acetate:n-hexane = 2:1): Rf = 0.48;

NMR: δ = 7.00-6.87(1H,m), 6.35-6.04(2H,m), 5.00-4.85(1H,m), 3.66(3H,s), 1.00-0.90(6H,m);

IR: ν = 3450, 2935, 1738, 1705, 1625, 1603 $cm^{-1}$;

Mass:m/e = 374, 343.

(b) (i) Methyl (13E)-(9α,15S)-6,11-dioxo-9,15-dihydroxy-15-(3-propylcyclopentyl)-16,17,18,19,20-pentanorprost-13-enoate [i.e. 15-(3-propylcyclopentyl)-16,17,18,19,20-pentanor-6-keto-$PGD_1$ methyl ester] and

(ii) Methyl (12EZ,14EZ)-(9α)-6,11-dioxo-9-hydroxy-15-(3-propylcyclopentyl)-16,17,18,19,20-pentanorprosta-12,14-dienoate

Starting material: 6,9-epoxy compound prepared in Reference Example 5(b);

Title compound (i):

TLC (ethyl acetate:n-hexane = 2:1): Rf = 0.20;

NMR: δ = 5.73-5.30(2H,m), 4.98-4.70(½H m), 4.60-4.50(½H,m), 4.10(1H,m), 3.68(3H,s), 0.88(3H,t);

IR: ν= 3450, 2935, 1732, 1716 $cm^{-1}$;

Mass:m/e = 404, 386, 373.

Title compound (ii):

TLC (ethyl acetate:n-hexane = 2:1): Rf = 0.48;

NMR: δ = 7.03-6.85(1H,m), 6.35-6.00(2H,m), 5.00-4.80(1H,m), 3.66(3H,s), 0.89(3H,t);

IR: ν= 3450, 2930, 1732, 1712, 1623, 1603 $cm^{-1}$;

Mass:m/e = 386, 355.

(c) (i) Methyl (13E)-(9α,15S)-6,11-dioxo-9,15-dihydroxy-16-phenyl-17,18,19,20-tetranorprost-13-enoate [i.e. 16-phenyl-17,18,19,20-tetranor-6-keto-$PGD_1$ methyl ester] and

(ii) Methyl (12EZ,14EZ)-(9α)-6,11-dioxo-9-hydroxy-16-phenyl-17,18,19,20-tetranorprosta-12,14-dienoate

Starting material: 6,9-epoxy compound prepared in Reference Example 5(c);

Title compound (i):

TLC (ethyl acetate:n-hexane = 2:1): Rf = 0.18;

NMR: $\delta$ = 7.5-7.1(5H,m), 5.70-5.34(2H,m), 4.90(½H,m), 4.52(½H,m), 3.66(3H,s).

IR: $\nu$ = 3470, 2940, 1736 $cm^{-1}$;

Mass:m/e = 384, 366, 353.

Title compound (ii):

TLC (ethyl acetate:n-hexane = 2:1): Rf = 0.46;

NMR: $\delta$ = 7.5-7.1(5H,m), 7.00-6.85(1H,m), 6.35-6.04(2H,m), 5.00-4.86(1H,m), 3.66(3H,s);

IR: $\nu$ = 3460, 2930, 1732, 1710, 1620 1600 $cm^{-1}$;

Mass:m/e = 366, 335.

(d) (i) Methyl (13<u>E</u>)-(9α,15<u>R</u>)-6,11-dioxo-9,15-dihydroxy-16-(3-chlorophenoxy)-17,18,19,20-tetranor-prost-13-enoate [i.e. 16-3-chlorophenoxy)-17,18,19,20-tetranor-6-keto-$PGD_1$ methyl ester] and

(ii) Methyl (12<u>EZ</u>,14<u>EZ</u>)-(9α)-6,11-dioxo-9-hydroxy-16-(3-chlorophenoxy)-17,18,19,20-tetranor-prosta-12,14-dienoate

Starting material: 6,9-epoxy compound prepared in Reference Example 5(d);

Title compound (i):

TLC (ethyl acetate:n-hexane = 2:1): Rf = 0.17;

NMR: $\delta$ = 7.3-6.7(4H,m), 5.7-5.3(2H,m), 4.86(½H,m), 4.56(½H,m), 3.95(2H,d), 3.68(3H,s);

IR: $\nu$ = 3450, 2930, 1730, 1716, 1600 $cm^{-1}$;

Mass:m/e = 434, 416, 403.

Title compound (ii):

TLC (ethyl acetate:n-hexane = 2:1): Rf = 0.45;

NMR:$\delta$ = 7.3-6.7(5H,m), 6.40-6.05(2H,m), 4.95(1H,m);

IR: $\nu$ = 3450, 2930, 1730, 1710, 1620, 1600 $cm^{-1}$;

Mass:m/e = 416, 385.

## Example 2

Methyl (9Z,12EZ,14EZ)-6,11-dioxoprosta-9,12,14-trienoate

0.5 ml. of 1N hydrochloric acid was added to a solution of 22 mg. of 6-keto-$PGD_1$ methyl ester (prepared in Example 1) in 0.5 ml. of tetrahydrofuran and the mixture was refluxed for 30 minutes. After cooling, the solution was diluted with ethyl acetate. The diluted solution was washed successively with a saturated aqueous solution of sodium bicarbonate and a saturated aqueous solution of sodium chloride, dried over anhydrous magnesium sulphate, and concentrated under reduced pressure. The residue was purified by column chromatography on silica gel using a mixture of

n-hexane and ethyl acetate as eluent, to obtain 5 mg. of the title compound having the following physical characteristics:

TLC (n-hexane:ethyl acetate = 1:1): Rf = 0.47;

NMR: δ = 7.70-7.52(1H,m), 7.00-6.90(1H,m), 6.45-6.06(3H,m), 4.06-3.92(1H,m), 3.69(3H,s). 3.10-2.90(1H,m), 1.0-0.8(3H,m);

IR: ν = 2930, 1735, 1710, 1690, 1630 $cm^{-1}$;

Mass:m/e = 346($M^+$), 315.

The following compounds were obtained by the same procedure as Example 2:

(a) Methyl (9Z,12EZ,14EZ)-(17S)-6,11-dioxo-17,20-dimethylprosta-9,12,14-trienoate

Starting material: 17S,20-dimethyl-6-keto-$PGD_1$ methyl ester prepared in Example 1(a);

TLC (n-hexane:ethyl acetate = 1:1): Rf = 0.48;

NMR: δ = 7.62-7.53(1H,m), 6.94(1H,d), 6.40-6.07(3H,m), 4.00(1H,m), 3.68(3H,s), 2.97(1H,dd), 1.00-0.90(6H m);

IR: ν = 2930, 1738, 1693, 1630 $cm^{-1}$;

Mass:m/e = 374($M^+$), 343.

(b) Methyl (19Z,12EZ,14EZ)-6,11-dioxo-15-(3-propylcyclopentyl)-16,17,18,19,20-pentanorprosta-9,12,14-trienoate

Starting material: 15-(3-propylcyclopentyl)-16,17,18,19,20-pentanor-6-keto-$PGD_1$ methyl ester prepared in Example 1(b);

TLC (n-hexane:ethyl acetate = 1:1): Rf = 0.48;

NMR: δ = 7.68-7.52(1H,m), 6.96(1H,d), 6.42-6.08(3H,m), 4.00(1H,m), 3.68(3H,s), 2.99(1H,dd), 0.89(3H,t);

IR: ν = 2932, 1736, 1710, 1692, 1632 $cm^{-1}$;

Mass:m/e = 386($M^+$), 355.

(c) Methyl (9Z,12EZ,14EZ)-6,11-dioxo-16-phenyl-17,18,19,20-tetranorprosta-9,12,14-trienoate

Starting material: 16-phenyl-17,18,19,20-tetranor-6-keto-$PGD_1$ methyl ester prepared in Example 1(c);

TLC (n-hexane:ethyl acetate = 1:1): Rf = 0.46;

NMR: δ = 7.54-6.90(7H,m), 6.40-6.03(3H,m), 4.95(1H,m);

IR: ν = 2930, 1736, 1710, 1690, 1630 $cm^{-1}$;

Mass:m/e = 366($M^+$), 335.

(d) Methyl (9Z,12EZ,14EZ)-6,11-dioxo-16-(3-chlorophenoxy)-17,18,19,20-tetranorprosta-9,12,14-trienoate

Starting material: 16-(3-chlorophenoxy)-17,18,19,20-tetranor-6-keto-$PGD_1$ methyl ester prepared in Example 1(d);

TLC (n-hexane:ethyl acetate = 1:1): Rf = 0.45;

NMR: δ = 7.60(1H,dd), 7.3-6.7(5H,m), 6.45-6.05(3H,m), 4.05(1H,m);

IR: ν = 2930, 1735, 1712, 1692, 1630, 1600 $cm^{-1}$;

Mass:m/e = 416($M^+$), 385.

The present invention includes within its scope pharmaceutical compositions which comprise at least one prostaglandin D derivative of general formula (I), cyclodextrin clathrate thereof or, when R represents a hydrogen atom, non-toxic salt thereof together with a pharmaceutical carrier or coating.

In clinical practice, for the prevention or the therapy (including therapy to secure remission) against leukemia or solid cancer, the compounds of the present invention will normally be administered systemically or partially; usually by oral or parenteral (e.g. intravenous, subcutaneous or intramuscular) administration.

Solid compositions for oral administration include compressed tablets, pills, dispersible powders and granules. In such solid compositions, one or more of the active compound(s) is, or are, admixed with at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, and disintegrating agents, such as cellulose calcium gluconate. The tablets or pills may, if desired, be made into enteric film-coated or gastric film-coated tablets or pills, such as sugar-coated, gelatin-coated,

hydroxypropylcellulose-coated or hydroxypropylmethylcellulose phthalate-coated tablets or pills; two or more layers may be used.

The compositions for oral administration also include capsules of absorbable material such as gelatin, containing one or more of the active substances with or without the addition of diluents or excipients.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise at least one compound of the present invention.

Preparations according to the invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such

as ethanol, and Tween 80 (registered Trade Mark). These compositions may also include adjuvants such as preserving, wetting, emulsifying and dispersing agents. They may be sterilised, for example, by filtration through a bacteria-retaining filter, by incorporation of sterilising agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal administration; and pessaries for vaginal administration. Such compositions are prepared by known methods.

The percentage of active ingredient in the compositions of the invention may be varied, it being necessary that it should constitute a proportion such that a suitable dosage for the therapeutic effect desired shall be obtained. Obviously several unit dosage forms may be administered at about the same time. In general, the preparations should normally contain at least 0.025% by weight of active substance when required for administration by injection; for oral administration the preparations will normally contain at least 0.1% by

weight of active substance.

The dose to be administered is determined depending upon, for example, age, body weight, symptoms, the desired therapeutic effects, the route of administration, and the duration of the treatment.

In the human adult, the doses per person are generally between 5 mg and 500 mg by oral administration, and between 500 μg and 50 mg by parenteral, preferably intravenous,administration for the prevention or the therapy (including therapy to secure remission) against leukemia or solid cancer, and can be administered up to several times per day.

As mentioned above, the doses to be used depend on various conditions. Therefore, there may be cases in which doses greater than the range specified above, or lower than the ranges specified above, may be used.

The following Examples illustrate pharmaceutical compositions according to the invention.

Example 3

A solution of 1 g. of 6-keto-$PGD_1$ methyl ester in 5 ml. of ethanol was mixed with 5 g. of microcrystalline cellulose, and the mixture was dried well. To the resulting mixture, 100 mg of magnesium stearate, 20 mg. of silicon dioxide, 10 mg. of talc and 200 mg. of cellulose calcium gluconate (CCG) were added and sufficient

microcrystalline cellulose was then added to obtain 10 g. of mixture. After mixing well, the mixture was punched out in conventional manner to obtain 100 tablets each containing 10 mg. of the active ingredient.

Example 4

α-and β-Cyclodextrin clathrate of 6-keto-$PGD_1$ methyl ester [which was prepared as follows; 2.4 g. of α-cyclodextrin and 1 g. of β-cyclodextrin were dissolved in 200 ml. of water. To the resulting solution, 100 mg. of 6-keto-$PGD_1$ methyl ester was added, and the solution was stirred well and then concentrated under reduced pressure to obtain the clathrate.] was dissolved in 150 ml.of distilled water for injection. The solution was sterilized by filtration in conventional manner, placed in 1.5 ml. portions in 5 ml. ampoules and freeze-dried to obtain 100 ampoules of solid composition for injection each containing 1 mg. of the active ingredient.

Example 5

Tablets and solid compositions for injection were prepared using the compounds of Example 1(ii), 1(a)(i), 1(a)(ii), 1(b)(i), 1(b)(ii), 1(c)(i), 1(c)(ii), 1(d)(i), 1(d)(ii), 2, 2(a), 2(b), 2(c) and 2(d), in the same manner as described in Examples 3 and 4.

**CLAIMS FOR CONTRACTING STATES: BE,CH,DE,FR,GB,IT,LI,LU, NL,SE**

1. A prostaglandin D derivative of the formula:

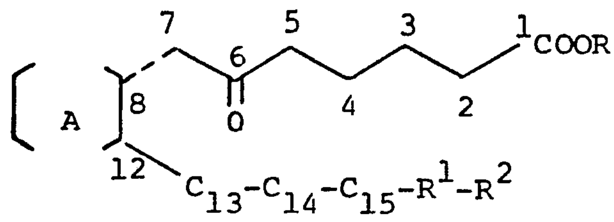

(I)

wherein [A] represents a group of the formula:

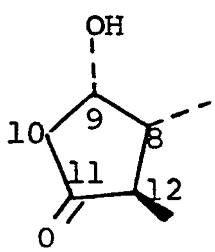

(II)

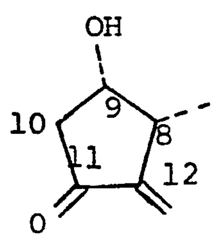

(III)

or

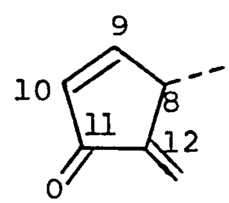

(IV)

$C_{13}-C_{14}-C_{15}$ represents

(i) a group of the formula:

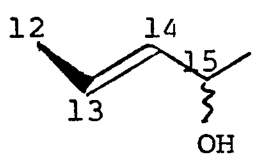

(V)

when [A] represents a group of the formula (II), or

(ii) a group of the formula:

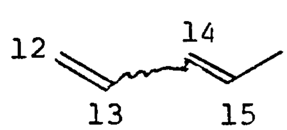

(VI)

when [A] represents a group of the formula (III) or (IV), R represents a hydrogen atom or a straight-chain or branched-chain alkyl group of 1 - 12 carbon atoms, $R^1$ represents a single bond or a straight-chain or branched-chain alkylene group of 1 - 5 carbon atoms, $R^2$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms, or represents a phenyl group or phenoxy group either unsubstituted or substituted by at least one halogen atom, trifluoromethyl group or straight-chain or branched-chain alkyl group of 1 - 4 carbon atoms, the double bond between $C_9$ - $C_{10}$ in the formula (IV) is Z, the double bond between $C_{13}$ - $C_{14}$ in formula (V) is E, and the double bonds between $C_{12}$ - $C_{13}$ and between $C_{14}$ - $C_{15}$ in formula (VI), which may be in the same or different configurations, are E,Z or a mixture thereof, with the proviso that when $R^1$ represents a single bond, $R^2$ does not represent a substituted or unsubstituted phenoxy group; and, when [A] represents a group of formula (II) or (III), mixtures of the prostaglandin derivatives of general formula (I) and their tautomers; and, when R represents a hydrogen atom, non-toxic salts thereof; and cyclodextrin clathrates thereof.

2. A prostaglandin derivative according to claim 1 wherein R is a hydrogen atom, a methyl group or an ethyl group.

3. A prostaglandin derivative according to claim 1 or 2 wherein the hydroxy group attached to the 15-position carbon atom of the group of formula (V) is in α-configuration.

4. A prostaglandin derivative according to any one of the preceding claims wherein the grouping $R^1-R^2$ represents a pentyl or hexyl group unsubstituted or substituted by one or two methyl groups; a cyclobutyl, cyclopentyl or cyclohexyl group unsubstituted or substituted by one methyl, ethyl, propyl or butyl group; or a phenyl or phenoxy group either unsubstituted or substituted by one chlorine atom, trifluoromethyl group, or methyl or ethyl group.

5. A prostaglandin derivative according to claim 1 which is 6-keto-$PGD_1$ methyl ester, 17<u>S</u>,20-dimethyl-6-keto-$PGD_1$ methyl ester, 15-(3-propylcyclopentyl)-16,17,18,19,20-pentanor-6-keto-$PGD_1$ methyl ester, 16-phenyl-17,18,19,20-tetranor-6-keto-$PGD_1$ methyl ester, 16-(3-chlorophenoxy)-17,18,19,20-tetranor-6-keto-$PGD_1$ methyl ester, methyl (12<u>EZ</u>,14<u>EZ</u>)-(9α)-6,11-dioxo-9-hydroxyprosta-12,14-dienoate, methyl (12<u>EZ</u>,14<u>EZ</u>)-(9α,17<u>S</u>)-6,11-dioxo-9-hydroxy-17,20-dimethylprosta-12,14-dienoate, methyl (12<u>EZ</u>,14<u>EZ</u>)-(9α)-6,11-dioxo-9-hydroxy-15-(3-propylcyclopentyl)-16,17,18,19,20-pentanorprosta-12,14-dienoate, methyl (12<u>EZ</u>,14<u>EZ</u>)-

(9α)-6,11-dioxo-9-hydroxy-16-phenyl-17,18,19,20-tetranorprosta-12,14-dienoate, methyl (12EZ,14EZ)-(9α)-6,11-dioxo-9-hydroxy-16-(3-chlorophenoxy)-17,18,19,20-tetranorprosta-12,14-dienoate, methyl (9Z,12EZ,14EZ)-6,11-dioxoprosta-9,12,14-trienoate, methyl (9Z,12EZ,14EZ)-(17S)-6,11-dioxo-17,20-dimethylprosta-9,12,14-trienoate, methyl (9Z,12EZ,14EZ)-6,11-dioxo-15-(3-propylcyclopentyl)-16,17,18,19,20-pentanorprosta-9,12,14-trienoate, methyl (9Z,12EZ,14EZ)-6,11-dioxo-16-phenyl-17,18,19,20-tetranorprosta-9,12,14-trienoate or methyl (9Z,12EZ,14EZ)-6,11-dioxo-16-(3-chlorophenoxy)-17,18,19,20-tetranorprosta-9,12,14-trienoate or a cyclodextrin clathrate thereof.

6. A process for the preparation of a prostaglandin derivative as claimed in claim 1 which comprises:

(i) when [A] represents a group of the formula (II) and $C_{13}$-$C_{14}$-$C_{15}$ represents a group of formula (V) or [A] represents a group of the formula (III) and $C_{13}$-$C_{14}$-$C_{15}$ represents a group of the formula (VI) and the other symbols are as defined in claim 1, the hydrolysis under acidic conditions of a compound of the general formula:

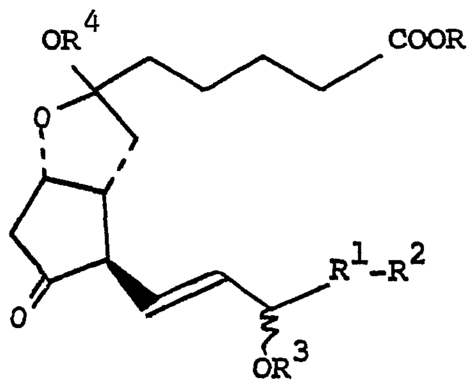

(VII)

(wherein $R^3$ represents a tetrahydropyran-2-yl group or tetrahydrofuran-2-yl group either unsubstituted or substituted by at least one alkyl group, or a 1-ethoxyethyl group, $R^4$ represents a lower alkyl group and the other symbols are as defined in claim 1, and the absolute configuration of the 6-position carbon atom is R or S or a mixture thereof, followed by separation of the mixture of prostaglandins obtained of the general formulae:

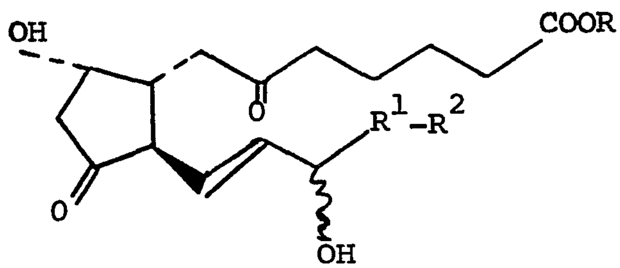

(Ia)

and

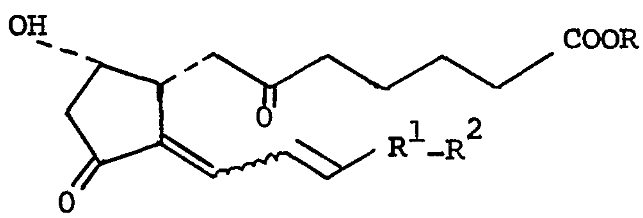

(Ib)

(wherein the various symbols are as defined in claim 1), or

(ii) when [A] represents a group of formula (IV), $C_{13}$-$C_{14}$-$C_{15}$ represents a group of formula (VI) and the other symbols are as defined in claim 1, reacting a prostaglandin derivative of general formula (Ia) or (Ib) (wherein the various symbols are as defined in claim 1) or a compound of general formula (VII) (wherein $R^3$ and $R^4$ are as hereinbefore defined and the other symbols are as defined in claim 1) with an aqueous acid, optionally followed by the step of converting a prostaglandin derivative of general formula (I) wherein R represents a hydrogen atom into an alkyl ester having from 1 to 12 carbon atoms in the alkyl radical or into a non-toxic salt, or by the step of converting a prostaglandin derivative of general formula (I) into a cyclodextrin clathrate thereof.

7. A pharmaceutical composition which comprises, as active ingredient, a prostaglandin derivative as claimed in claim 1, or a cyclodextrin clathrate thereof, or, when R in general formula I depicted in claim 1 represents a hydrogen atom, a non-toxic salt thereof, in association with a pharmaceutical carrier or coating.

8. A prostaglandin derivative as claimed in claim 1, or cyclodextrin clathrate thereof or, when R in general formula I represents a hydrogen atom, non-toxic salt thereof, for use in the prevention or therapy of leukemia or solid cancer.

9. A compound of general formula (VII) depicted in claim 6, wherein $R^3$ and $R^4$ are as defined in claim 6 and the other symbols are as defined in claim 1.

CLAIMS FOR CONTRACTING STATE: AT

1. A process for the preparation of a prostaglandin D derivative of the general formula:

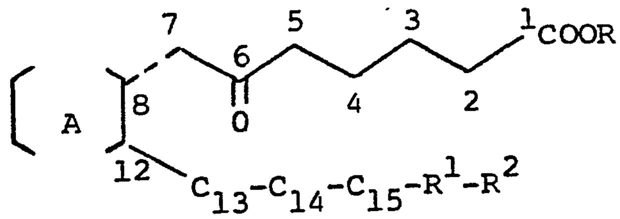

(I)

wherein [A] represents a group of the formula:

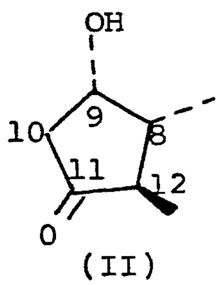

(II)

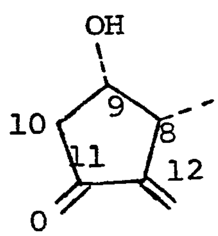

(III)

or

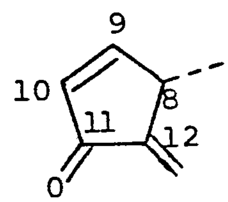

(IV)

$C_{13}-C_{14}-C_{15}$ represents

(i) a group of the formula:

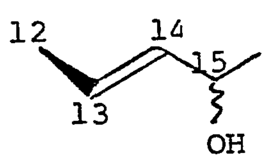

(V)

when [A] represents a group of the formula (II), or

(ii) a group of the formula:

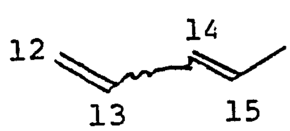

(VI)

when [A] represents a group of the formula (III) or (IV), R represents a hydrogen atom or a straight-chain or branched-chain alkyl group of 1 - 12 carbon atoms, $R^1$ represents a single bond or a straight-chain or branched-chain alkylene group of 1 - 5 carbon atoms, $R^2$ represents a straight-chain or branched-chain alkyl group of 1 - 8 carbon atoms, a cycloalkyl group of 4 - 7 carbon atoms either unsubstituted or substituted by at least one straight-chain or branched-chain alkyl group of 1 - 8 carbon atoms, or represents a phenyl group or phenoxy group either unsubstituted or substituted by at least one halogen atom, trifluoromethyl group or straight-chain or branched-chain alkyl group of 1 - 4 carbon atoms, the double bond between $C_9$ - $C_{10}$ in the formula (IV) is Z, the double bond between $C_{13}$ - $C_{14}$ in formula (V) is E, and the double bonds between $C_{12}$ - $C_{13}$ and between $C_{14}$ - $C_{15}$ in formula (VI), which may be in the same or different configurations, are E,Z or a mixture thereof, with the proviso that when $R^1$ represents a single bond, $R^2$ does not represent a substituted or unsubstituted phenoxy group; and, when [A] represents a group of formula (II) or (III), mixtures of the prostaglandin derivatives of general formula (I) and their tautomers; and, when R represents a hydrogen atom, non-toxic salts thereof; and cyclodextrin clathrates thereof, which comprises:

(i) when [A] represents a group of the formula (II) and

$C_{13}-C_{14}-C_{15}$ represents a group of formula (V) or [A] represents a group of the formula (III) and $C_{13}-C_{14}-C_{15}$ represents a group of the formula (VI), and the other symbols are as hereinbefore defined, the hydrolysis under acidic conditions of a compound of the general formula:

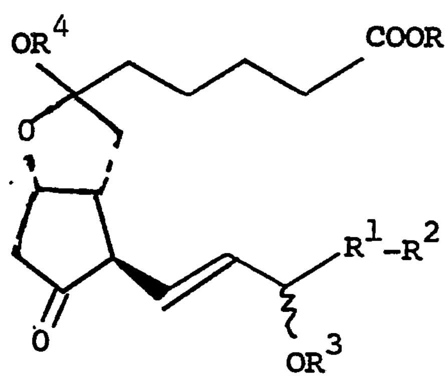

(VII)

(wherein $R^3$ represents a tetrahydropyran-2-yl group or tetrahydrofuran-2-yl group either unsubstituted or substituted by at least one alkyl group, or a 1-ethoxy-ethyl group, $R^4$ represents a lower alkyl group and the other symbols are as hereinbefore defined, and the absolute configuration of the 6-position carbon atom is R or S or a mixture thereof), followed by the separation of the mixture of prostaglandins obtained of the general formulae:

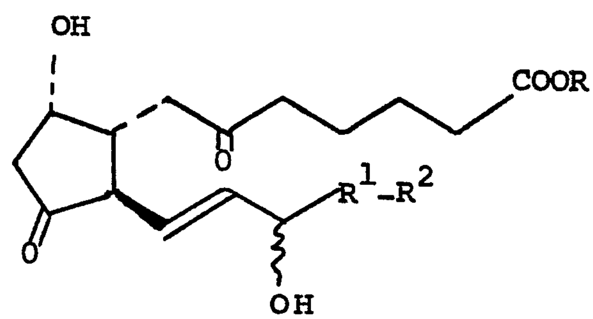

(Ia)

and

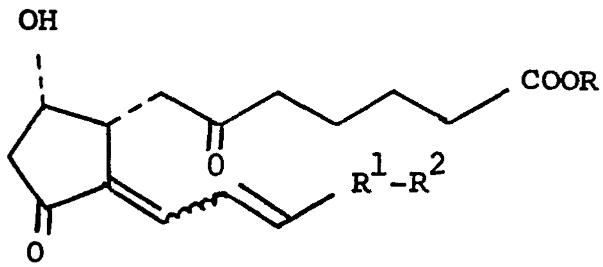

(Ib)

(wherein the various symbols are as hereinbefore defined), or

(ii) when [A] represents a group of the formula (IV), $C_{13}-C_{14}-C_{15}$ represents a group of the formula (VI) and the other symbols are as hereinbefore defined, reacting a prostaglandin derivative of general formula (Ia) or (Ib) (wherein the various symbols are as hereinbefore defined)or a compound of general formula (VII) (wherein the various symbols are as hereinbefore defined) with an aqueous acid, optionally followed by the step of converting a prostaglandin derivative of general formula (I) wherein R represents a hydrogen atom into an alkyl ester having from 1 to 12 carbon atoms in the alkyl radical or into a non-toxic salt, or by the step of converting a prostaglandin derivative of general formula (I) into a cyclodextrin clathrate thereof.

2. A process according to claim 1 wherein $R^3$ represents a tetrahydropyran-2-yl group.

3. A process according to claim 1 wherein $R^4$ represents a methyl group.

4. A process according to claim 1 or 2 wherein the hydrolysis under acidic conditions of a compound of general formula (VII) is carried out using a mixture of dilute hydrochloric acid and tetrahydrofuran, a mixture of dilute hydrochloric acid and methanol, a mixture of acetic acid, water and tetrahydrofuran, a mixture of phosphoric acid, water and tetrahydrofuran, or a mixture of p-toluenesulphonic acid and anhydrous methanol.

5. A process according to claim 1, 2 or 3 wherein the reaction of a prostaglandin derivative of general formula (Ia) or (Ib) or a compound of general formula (VII) with an aqueous acid is carried out in tetrahydrofuran using 1N hydrochloric acid at the reflux temperature of the reaction mixture.